# EUROPEAN PATENT APPLICATION

(11) **EP 4 400 171 A2**
(43) Date of publication of application: **17.07.2024**
(21) Application number: 24163571.3
(22) Date of filing: 01.12.2017
(51) Int. Cl.: A61P 25/18

(54) **USE OF VALBENAZINE FOR TREATING SCHIZOPHRENIA OR SCHIZOAFFECTIVE DISORDER**

(30) Priority: 02.12.2016 US 201662429652 P
(62) Divisional of application: 17817594.9
(71) Applicant: NEUROCRINE BIOSCIENCES, INC., San Diego, CA 92130-1102 (US)
(72) Inventor: O'BRIEN, Christopher, F., San Diego, 92130 (US); GRIGORIADIS, Dimitri, E., San Diego, 92130 (US)
(74) Representative: Mewburn Ellis LLP

(57) **Abstract**

Provided herein are methods for treating schizophrenia or schizoaffective disorder by administering to a subject in need thereof (S)-2-amino-3-methyl-butyric acid (2R,3R,1 1bR)-3-isobutyl-9,10-dimethoxy-1,3,4,6,7,11b-hexahydro-2H-pyrido[2,I-a]isoquinolin-2-yl ester, or an isotopic variant thereof, or a pharmaceutically acceptable salt or polymorph thereof.

## Description

### FIELD

Provided herein are methods for treating schizophrenia or schizoaffective disorder by administering to a subject in need thereof (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester, or an isotopic variant thereof; or a pharmaceutically acceptable salt or polymorph thereof.

### BACKGROUND

Schizophrenia affects approximately 1% of the adult population and reduces life expectancy by an average of 20 to 25 years through the impact of the disorder on self-care and physical health, as well as through suicide. At the present time the etiological mechanisms underlying schizophrenia are poorly understood. Schizophrenia is diagnosed clinically, based on characteristic symptoms of psychosis, disorganization and so called 'negative' symptoms (representing a reduced range of emotional expression, reduced production of speech and a lack of volition/motivation); duration of illness; impaired functioning; and the exclusion of other disorders such as autism and bipolar disorder. For clinicians, identifying which psychotic patients have schizophrenia requires clinical acumen and familiarity with the DSM-IV or ICD-10 diagnostic manuals (*see, e.g.,* Corvin, *BMC Biol.* 2011; 9: 77).

Antipsychotic drug therapy is a pillar in the treatment of schizophrenia. These antipsychotic drugs, also known as neuroleptics, generally cause a reduction of the 'positive' symptoms of schizophrenia, namely psychosis, thought disorders, and disorganized behavior. Antipsychotics generally have a lesser influence on cognition and on the 'negative' symptoms of the disease, which include lack of motivation and emotion, social withdrawal, lack of interest in everyday activities, and the reduced ability to plan or carry out activities.

First generation or "typical" antipsychotics have been used for over 50 years in the treatment of schizophrenia and other psychotic disorders. The first marketed antipsychotic was chlorpromazine; other typical antipsychotics include fluphenazine, haloperidol, loxapine, molindone, perphenazine, pimozide, sulpiride, thioridazine, and trifluoperazine. These typical antipsychotics all gain their primary efficacy through D2 dopamine receptor antagonism and have a propensity to cause movement disorders including parkinsonism (tremor, rigidity, bradykinesia and gait instability) as well as dystonia, dyskinesia (e.g., tardive dyskinesia), and akathisia.

Second generation or "atypical" antipsychotics were developed, and these drugs possess a lower risk of causing TD and related movement disorders with chronic administration. These drugs include aripiprazole, asenapine, clozapine, iloperidone, olanzapine, paliperidone, quetiapine, risperidone, and ziprasidone. These atypical antipsychotics all exert their primary efficacy through D2 dopamine receptor antagonism with additional effects on receptors for other neurotransmitters. These atypical antipsychotics are associated with metabolic side effects sufficient to affect life expectancy. These side effects include a propensity to induce weight gain, as well as related metabolic disturbances such as hypertriglyceridemia and hyperglycemia. Clozapine appears to be the most effective as treatment for severe mental illness, but it has additional serious medical side effects, including a significant incidence of agranulocytosis that requires frequent monitoring of patients' white blood counts as a requirement for using the drug.

In addition to treatment of schizophrenia or schizoaffective disorder, certain antipsychotic medications have been approved as treatments of bipolar disorder, major depressive disorder (MDD), and autism spectrum disorders. Off-label use is prevalent, particularly of atypicals, which are used for the treatment of various conditions including anxiety, attention-deficit hyperactivity disorder (ADHD), dementia, depression, insomnia, obsessive-compulsive disorder (OCD), post-traumatic stress disorder, substance abuse, and Tourette's syndrome.

Because the side effects associated with administration of antipsychotic medications can significantly impact a patient's health and well-being, alternatives to the current therapies are needed.

The reversible inhibition of the vesicular monoamine transporter-2 system (VMAT2) by 3-isobutyl-9,10-dimethoxy-1,3,4,6,7,11b-hexahydro-2H-pyrido[2,l-a]isoquinolin-2-one, also known as tetrabenazine (TBZ), improves the treatment of various neurological disorders. However, the drawbacks to such treatment are the fluctuating response, the need for frequent intake due to TBZ rapid metabolism, and side effects. Side effects associated with TBZ include sedation, depression, akathisia, and parkinsonism.

TBZ, which contains two chiral centers and is a racemic mix of two stereoisomers, is rapidly and extensively metabolized in vivo to its reduced form, 3-isobutyl-9, 10-dimethoxy-1,3,4,6,7,11b-hexahydro-2H-pyrido[2,l-a]isoquinolin-2-ol, also known as dihydrotetrabenazine (DHTBZ). DHTBZ is thought to exist as four individual isomers: (±) alpha-DHTBZ and (±) beta-DHTBZ. The (2R, 3R, 11bR) or (+) alpha-DHTBZ is believed to be the absolute configuration of the active metabolite (Kilbourn *et al.*, *Chirality*, 1997, 9, 59-62). Tetrabenazine is approved in certain European countries for therapy of chorea in patients with Hungtington's disease. However, tetrabenazine is rapidly metabolized and must frequently be administered throughout the day. (Muller, Expert Opin. Investig. Drugs, 2015, 24, 737-742). Therefore, there is an unmet need in the art to develop effective therapeutics for treatment of neurological disorders, including schizophrenia or schizoaffective disorder.

Valbenazine, (*S*)-2-amino-3-methyl-butyric acid (2*R*,3*R*,11*bR*)-3-isobutyl-9,10-dimethoxy-1,3,4,6,7,11b-hexahydro-2H-pyrido[2,l-a]isoquinolin-2-yl ester, the purified prodrug of the (+)-α-isomer of dihydrotetrabenazine, is also an inhibitor of the vesicular monoamine transporter-2 system (VMAT2).

### SUMMARY OF THE DISCLOSURE

Provided herein are methods for treating schizophrenia or schizoaffective disorder by administering to a subject in need thereof a VMAT2 inhibitor, or a pharmaceutical composition comprising the VMAT2 inhibitor.

Also provided herein are methods of treating schizophrenia or schizoaffective disorder by administering (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof.

Also provided herein is a pharmaceutical composition for use in treating schizophrenia or schizoaffective disorder, comprising (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof.

### BRIEF DESCRIPTION OF THE FIGURES

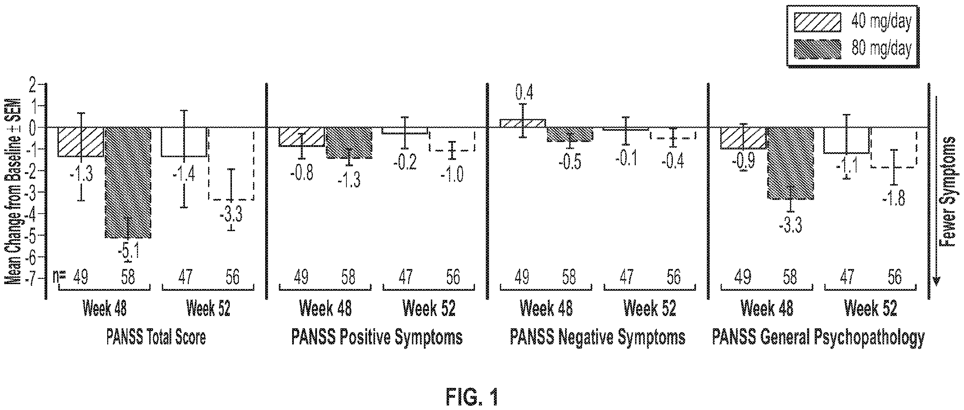

FIG. 1 represent the mean changes in scores from baseline as measured by the Positive and Negative Syndrome Scale (PANSS) after long-term valbenazine treatment (Week 48) and treatment withdrawal (Week 52). PANSS was administered only to subjects with schizophrenia/schizoaffective disorder (40 mg, n=154; 80 mg, n=155).

### DETAILED DESCRIPTION OF THE INVENTION

### Definitions

To facilitate understanding of the disclosure set forth herein, a number of terms are defined below.

Generally, the nomenclature used herein and the laboratory procedures in organic chemistry, medicinal chemistry, and pharmacology described herein are those well known and commonly employed in the art. Unless defined otherwise, all technical and scientific terms used herein generally have the same meaning as commonly understood by one of ordinary skill in the art to which this disclosure belongs.

The term "subject" refers to an animal, including, but not limited to, a primate (e.g., human), cow, pig, sheep, goat, horse, dog, cat, rabbit, rat, or mouse. The terms "subject" and "patient" are used interchangeably herein in reference, for example, to a mammalian subject, such as a human subject, in one embodiment, a human.

As used herein, "isotopically enriched" refers to an atom having an isotopic composition other than the natural isotopic composition of that atom. "Isotopically enriched" may also refer to a compound containing at least one atom having an isotopic composition other than the natural isotopic composition of that atom.

With regard to the compounds provided herein, when a particular atomic position is designated as having deuterium or "D," it is understood that the abundance of deuterium at that position is substantially greater than the natural abundance of deuterium, which is about 0.015%. A position designated as having deuterium typically has a minimum isotopic enrichment factor of, in particular embodiments, at least 1000 (15% deuterium incorporation), at least 2000 (30% deuterium incorporation), at least 3000 (45% deuterium incorporation), at least 3500 (52.5% deuterium incorporation), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation) at each designated deuterium position.

The isotopic enrichment of the compounds provided herein can be determined using conventional analytical methods known to one of ordinary skill in the art, including mass spectrometry, nuclear magnetic resonance spectroscopy, and crystallography.

Isotopic enrichment (for example, deuteration) of pharmaceuticals to improve pharmacokinetics ("PK"), pharmacodynamics ("PD"), and toxicity profiles, has been demonstrated previously with some classes of drugs. *See,* for example, Lijinsky et. al., Food Cosmet. Toxicol., 20: 393 (1982); Lijinsky et. al., J. Nat. Cancer Inst., 69: 1127 (1982); Mangold et. al., Mutation Res. 308: 33 (1994); Gordon et. al., Drug Metab. Dispos., 15: 589 (1987); Zello et. al., Metabolism, 43: 487 (1994); Gately et. al., J. Nucl. Med., 27: 388 (1986); Wade D, Chem. Biol. Interact. 117: 191 (1999).

Isotopic enrichment of a drug can be used, for example, to (1) reduce or eliminate unwanted metabolites, (2) increase the half-life of the parent drug, (3) decrease the number of doses needed to achieve a desired effect, (4) decrease the amount of a dose necessary to achieve a desired effect, (5) increase the formation of active metabolites, if any are formed, and/or (6) decrease the production of deleterious metabolites in specific tissues and/or create a more effective drug and/or a safer drug for combination therapy, whether the combination therapy is intentional or not.

Replacement of an atom for one of its isotopes often will result in a change in the reaction rate of a chemical reaction. This phenomenon is known as the Kinetic Isotope Effect ("KIE"). For example, if a C-H bond is broken during a rate-determining step in a chemical reaction (*i.e.* the step with the highest transition state energy), substitution of a deuterium for that hydrogen will cause a decrease in the reaction rate and the process will slow down. This phenomenon is known as the Deuterium Kinetic Isotope Effect ("DKIE"). (*See, e.g.,* Foster et al., Adv. Drug Res., vol. 14, pp. 1-36 (1985); Kushner et al., Can. J. Physiol. Pharmacol., vol. 77, pp. 79-88 (1999)).

The magnitude of the DKIE can be expressed as the ratio between the rates of a given reaction in which a C-H bond is broken, and the same reaction where deuterium is substituted for hydrogen. The DKIE can range from about 1 (no isotope effect) to very large numbers, such as 50 or more, meaning that the reaction can be fifty, or more, times slower when deuterium is substituted for hydrogen. High DKIE values may be due in part to a phenomenon known as tunneling, which is a consequence of the uncertainty principle. Tunneling is ascribed to the small mass of a hydrogen atom, and occurs because transition states involving a proton can sometimes form in the absence of the required activation energy. Because deuterium has more mass than hydrogen, it statistically has a much lower probability of undergoing this phenomenon.

Tritium ("T") is a radioactive isotope of hydrogen, used in research, fusion reactors, neutron generators and radiopharmaceuticals. Tritium is a hydrogen atom that has 2 neutrons in the nucleus and has an atomic weight close to 3. It occurs naturally in the environment in very low concentrations, most commonly found as T₂O. Tritium decays slowly (half-life = 12.3 years) and emits a low energy beta particle that cannot penetrate the outer layer of human skin. Internal exposure is the main hazard associated with this isotope, yet it must be ingested in large amounts to pose a significant health risk. As compared with deuterium, a lesser amount of tritium must be consumed before it reaches a hazardous level. Substitution of tritium ("T") for hydrogen results in yet a stronger bond than deuterium and gives numerically larger isotope effects. Similarly, substitution of isotopes for other elements, including, but not limited to, ¹³C or ¹⁴C for carbon, ³³S, ³⁴S, or ³⁶S for sulfur, ¹⁵N for nitrogen, and ¹⁷O or ¹⁸O for oxygen, may lead to a similar kinetic isotope effect.

For example, the DKIE was used to decrease the hepatotoxicity of halothane by presumably limiting the production of reactive species such as trifluoroacetyl chloride. However, this method may not be applicable to all drug classes. For example, deuterium incorporation can lead to metabolic switching. The concept of metabolic switching asserts that xenogens, when sequestered by Phase I enzymes, may bind transiently and re-bind in a variety of conformations prior to the chemical reaction (e.g., oxidation). This hypothesis is supported by the relatively vast size of binding pockets in many Phase I enzymes and the promiscuous nature of many metabolic reactions. Metabolic switching can potentially lead to different proportions of known metabolites as well as altogether new metabolites. This new metabolic profile may impart more or less toxicity.

The animal body expresses a variety of enzymes for the purpose of eliminating foreign substances, such as therapeutic agents, from its circulation system. Examples of such enzymes include the cytochrome P450 enzymes ("CYPs"), esterases, proteases, reductases, dehydrogenases, and monoamine oxidases, to react with and convert these foreign substances to more polar intermediates or metabolites for renal excretion. Some of the most common metabolic reactions of pharmaceutical compounds involve the oxidation of a carbon-hydrogen (C-H) bond to either a carbon-oxygen (C-O) or carbon-carbon (C-C) pi-bond. The resultant metabolites may be stable or unstable under physiological conditions, and can have substantially different pharmacokinetic, pharmacodynamic, and acute and long-term toxicity profiles relative to the parent compounds. For many drugs, such oxidations are rapid. These drugs therefore often require the administration of multiple or high daily doses.

Therefore, isotopic enrichment at certain positions of a compound provided herein may produce a detectable KIE that will affect the pharmacokinetic, pharmacologic, and/or toxicological profiles of a compound provided herein in comparison with a similar compound having a natural isotopic composition.

The term "isotopic variant" refers to a therapeutic agent that contains an unnatural proportion of an isotope at one or more of the atoms that constitute such a therapeutic agent. In certain embodiments, an "isotopic variant" of a therapeutic agent contains unnatural proportions of one or more isotopes, including, but not limited to, hydrogen (¹H), deuterium (²H), tritium (³H), carbon-11 (¹¹C), carbon-12 (¹²C), carbon-13 (¹³C), carbon-14 (¹⁴C), nitrogen-13 (¹³N), nitrogen-14 (¹⁴N), nitrogen-15 (¹⁵N), oxygen-14 (¹⁴O), oxygen-15 (¹⁵O), oxygen-16 (¹⁶O), oxygen-17 (¹⁷O), oxygen-18 (¹⁸O), fluorine-17 (¹⁷F), fluorine-18 (¹⁸F), phosphorus-31 (³¹P), phosphorus-32 (³²P), phosphorus-33 (³³P), sulfur-32 (³²S), sulfur-33 (³³S), sulfur-34 (³⁴S), sulfur-35 (³⁵S), sulfur-36 (³⁶S), chlorine-35 (³⁵Cl), chlorine-36 (³⁶Cl), chlorine-37 (³⁷Cl), bromine-79 (⁷⁹Br), bromine-81 (⁸¹Br), iodine 123 (¹²³I), iodine-125 (¹²⁵I), iodine-127 (¹²⁷I), iodine-129 (¹²⁹I), and iodine-131 (¹³¹I). In certain embodiments, an "isotopic variant" of a therapeutic agent contains unnatural proportions of one or more isotopes, including, but not limited to, hydrogen (¹H), deuterium (²H), tritium (³H), carbon-11 (¹¹C), carbon-12 (¹²C), carbon-13 (¹³C), carbon-14 (¹⁴C), nitrogen-13 (¹³N), nitrogen-14 (¹⁴N), nitrogen-15 (¹⁵N), oxygen-14 (¹⁴O), oxygen-15 (¹⁵O), oxygen-16 (¹⁶O), oxygen-17 (¹⁷O), oxygen-18 (¹⁸O), fluorine-17 (¹⁷F), fluorine-18 (¹⁸F), phosphorus-31 (³¹P), phosphorus-32 (³²P), phosphorus-33 (³³P), sulfur-32 (³²S), sulfur-33 (³³S), sulfur-34 (³⁴S), sulfur-35 (³⁵S), sulfur-36 (³⁶S), chlorine-35 (³⁵Cl), chlorine-36 (³⁶Cl), chlorine-37 (³⁷Cl), bromine-79 (⁷⁹Br), bromine-81 (⁸¹Br), iodine 123 (¹²³I), iodine-125 (¹²⁵I), iodine-127 (¹²⁷I), iodine-129 (¹²⁹I), and iodine-131 (¹³¹I).

It will be understood that, in a therapeutic agent, any hydrogen can be ²H, for example, or any carbon can be ¹³C, for example, or any nitrogen can be ¹⁵N, for example, or any oxygen can be ¹⁸O, for example, where feasible according to the judgment of one of skill. In certain embodiments, an "isotopic variant" of a therapeutic agent contains unnatural proportions of deuterium (D).

The terms "treat," "treating," and "treatment" are meant to include alleviating or abrogating a disorder, disease, or condition, or one or more of the symptoms associated with the disorder, disease, or condition; or alleviating or eradicating the cause(s) of the disorder, disease, or condition itself.

The terms "prevent," "preventing," and "prevention" are meant to include a method of delaying and/or precluding the onset of a disorder, disease, or condition, and/or its attendant symptoms; barring a subject from acquiring a disorder, disease, or condition; or reducing a subject's risk of acquiring a disorder, disease, or condition.

As used herein, and unless otherwise specified, the terms "manage," "managing" and "management" refer to preventing or slowing the progression, spread or worsening of a disease or disorder, or of one or more symptoms thereof. Often, the beneficial effects that a subject derives from a prophylactic and/or therapeutic agent do not result in a cure of the disease or disorder. In this regard, the term "managing" encompasses treating a subject who had suffered from the particular disease in an attempt to prevent or minimize the recurrence of the disease.

As used herein, amelioration of the symptoms of a particular disorder by administration of a particular pharmaceutical composition refers to any lessening, whether permanent or temporary, lasting or transient, that can be attributed to or associated with administration of the composition.

The term "disorder" as used herein is intended to be generally synonymous, and is used interchangeably with, the terms "disease," "syndrome," and "condition" (as in medical condition), in that all reflect an abnormal condition of the human or animal body or of one of its parts that impairs normal functioning, is typically manifested by distinguishing signs and symptoms.

The term "therapeutically effective amount" are meant to include the amount of a compound that, when administered, is sufficient to prevent development of, or alleviate to some extent, one or more of the symptoms of the disorder, disease, or condition being treated. The term "therapeutically effective amount" also refers to the amount of a compound that is sufficient to elicit the biological or medical response of a biological molecule (e.g., a protein, enzyme, RNA, or DNA), cell, tissue, system, animal, or human, which is being sought by a researcher, veterinarian, medical doctor, or clinician.

As used herein, and unless otherwise specified, a "prophylactically effective amount" of a compound is an amount sufficient to prevent a disease or disorder, or prevent its recurrence. A prophylactically effective amount of a compound means an amount of therapeutic agent, alone or in combination with one or more other agent(s), which provides a prophylactic benefit in the prevention of the disease. The term "prophylactically effective amount" can encompass an amount that improves overall prophylaxis or enhances the prophylactic efficacy of another prophylactic agent.

The term "pharmaceutically acceptable carrier," "pharmaceutically acceptable excipient," "physiologically acceptable carrier," or "physiologically acceptable excipient" refers to a pharmaceutically-acceptable material, composition, or vehicle, such as a liquid or solid filler, diluent, solvent, or encapsulating material. In one embodiment, each component is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. *See,* Remington: The Science and Practice of Pharmacy, 22nd ed.; Pharmaceutical Press: 2012; Handbook of Pharmaceutical Excipients, 7th ed.; Rowe et al., Eds.; The Pharmaceutical Press: 2012; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, FL, 2009.

The term "dosage," "dosage unit," and "dosage level" are used interchangeably throughout the disclosure unless the context clearly dictates otherwise.

As used in the specification and the accompanying claims, the indefinite articles "a" and "an" and the definite article "the" include plural as well as singular referents, unless the context clearly dictates otherwise.

The term "about" or "approximately" means an acceptable error for a particular value as determined by one of ordinary skill in the art, which depends in part on how the value is measured or determined. In certain embodiments, the term "about" or "approximately" means within 1, 2, 3, or 4 standard deviations. In certain embodiments, the term "about" or "approximately" means within 30%, 25%, 20%, 15%, 10%, 9%, 8%, 7%, 6%, 5%, 4%, 3%, 2%, 1 %, 0.5%, or 0.05% of a given value or range.

The terms "active ingredient" and "active substance" refer to a compound, which is administered, alone or in combination with one or more pharmaceutically acceptable excipients, to a subject for treating, preventing, or ameliorating one or more symptoms of a disorder, disease, or condition. As used herein, "active ingredient" and "active substance" may be an optically active isomer or an isotopic variant of a compound described herein.

The terms "drug," "therapeutic agent," and "chemotherapeutic agent" refer to a compound, or a pharmaceutical composition thereof, which is administered to a subject for treating, preventing, or ameliorating one or more symptoms of a disorder, disease, or condition.

The term "solvate" refers to a complex or aggregate formed by one or more molecules of a solute, e.g., a compound provided herein, and one or more molecules of a solvent, which present in stoichiometric or non-stoichiometric amount. Suitable solvents include, but are not limited to, water, methanol, ethanol, n-propanol, isopropanol, and acetic acid. In certain embodiments, the solvent is pharmaceutically acceptable. In one embodiment, the complex or aggregate is in a crystalline form. In another embodiment, the complex or aggregate is in a noncrystalline form. Where the solvent is water, the solvate is a hydrate. Examples of hydrates include, but are not limited to, a hemihydrate, monohydrate, dihydrate, trihydrate, tetrahydrate, and pentahydrate.

As used herein and unless otherwise indicated, the terms "polymorph" and "polymorphic form" refer to solid crystalline forms of a compound or complex. Different polymorphs of the same compound can exhibit different physical, chemical and/or spectroscopic properties. Different physical properties include, but are not limited to stability (e.g., to heat or light), compressibility and density (important in formulation and product manufacturing), and dissolution rates (which can affect bioavailability). Differences in stability can result from changes in chemical reactivity (e.g., differential oxidation, such that a dosage form discolors more rapidly when comprised of one polymorph than when comprised of another polymorph) or mechanical characteristics (e.g., tablets crumble on storage as a kinetically favored polymorph converts to thermodynamically more stable polymorph) or both (e.g., tablets of one polymorph are more susceptible to breakdown at high humidity). Different physical properties of polymorphs can affect their processing. For example, one polymorph might be more likely to form solvates or might be more difficult to filter or wash free of impurities than another due to, for example, the shape or size distribution of particles of it.

Polymorphs of a molecule can be obtained by a number of methods known in the art. Such methods include, but are not limited to, melt recrystallization, melt cooling, solvent recrystallization, desolvation, rapid evaporation, rapid cooling, slow cooling, vapor diffusion and sublimation. Polymorphs can be detected, identified, classified and characterized using well-known techniques such as, but not limited to, differential scanning calorimetry (DSC), thermogravimetry (TGA), X-ray powder diffractometry (XRPD), single crystal X-ray diffractometry, vibrational spectroscopy, solution calorimetry, solid state nuclear magnetic resonance (NMR), infrared (IR) spectroscopy, Raman spectroscopy, hot stage optical microscopy, scanning electron microscopy (SEM), electron crystallography and quantitative analysis, particle size analysis (PSA), surface area analysis, solubility, and rate of dissolution.

The term "crystalline form" of a compound can refer to any crystalline form of the compound as a free acid, the compound as a free base, as an acid addition salt of the compound, an base addition salt of the compound, a complex of the compound, a solvate (including hydrate) of the compound, or a co-crystal of the compound. The term "solid form" of a compound can refer to any crystalline form of the compound or any amorphous form of the compound as a free acid, the compound as a free base, as an acid addition salt of the compound, an base addition salt of the compound, a complex of the compound, or a solvate (including hydrate) of the compound, or a co-precipitation of the compound. In many instances, the terms "crystalline form" and "solid form" can refer to those that are pharmaceutically acceptable, including, for example, those of pharmaceutically acceptable addition salts, pharmaceutically acceptable complexes, pharmaceutically acceptable solvates, pharmaceutically acceptable co-crystals, and pharmaceutically acceptable co-precipitations.

The term "schizophrenia or schizoaffective disorder " includes but is not limited to illnesses that describe abnormal social behavior and failure to understand what is real. Schizophrenia is described in terms of positive and negative (or deficit) symptoms. Positive symptoms are those that most individuals do not normally experience, but are present in people with schizophrenia. They can include delusions, disordered thoughts and speech, and tactile, auditory, visual, olfactory and gustatory hallucinations, typically regarded as manifestations of psychosis. Hallucinations are also typically related to the content of the delusional theme. Negative symptoms are deficits of normal emotional responses or of other thought processes. They commonly include flat expressions or little emotion, poverty of speech, inability to experience pleasure, lack of desire to form relationships, and lack of motivation.

The term "VMAT2" refers to human vesicular monoamine transporter isoform 2, an integral membrane protein that acts to transport monoamines, particularly neurotransmitters such as dopamine, norepinephrine, serotonin, and histamine, from cellular cytosol into synaptic vesicles.

The term "VMAT2 inhibitor", "inhibit VMAT2", or "inhibition of VMAT2" refers to the ability of a compound disclosed herein to alter the function of VMAT2. A VMAT2 inhibitor may block or reduce the activity of VMAT2 by forming a reversible or irreversible covalent bond between the inhibitor and VMAT2 or through formation of a noncovalently bound complex. Such inhibition may be manifest only in particular cell types or may be contingent on a particular biological event. The term "VMAT2 inhibitor", "inhibit VMAT2", or "inhibition of VMAT2" also refers to altering the function of VMAT2 by decreasing the probability that a complex forms between a VMAT2 and a natural substrate. In some embodiments, modulation of the VMAT2 may be assessed using the method described in WO 2005077946; WO 2008/058261; EP 1716145; Kilbourn et al., European Journal of Pharmacology 1995, (278), 249-252; Lee et al., J. Med. Chem. , 1996, (39), 191-196; Scherman et al., Journal of Neurochemistry 1988, 50(4), 1131-36; Kilbourn et al., Synapse 2002, 43(3), 188-194; Kilbourn et al., European Journal of Pharmacology 1997, 331(2-3), 161-68; and Erickson et al., Journal of Molecular Neuroscience 1995, 6(4), 277-87.

"Pharmaceutically acceptable salt" refers to any salt of a compound provided herein which retains its biological properties and which is not toxic or otherwise undesirable for pharmaceutical use. Such salts may be derived from a variety of organic and inorganic counter-ions well known in the art. Such salts include, but are not limited to: (1) acid addition salts formed with organic or inorganic acids such as hydrochloric, hydrobromic, sulfuric, nitric, phosphoric, sulfamic, acetic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, tert-butylacetic, lauryl sulfuric, gluconic, benzoic, glutamic, hydroxynaphthoic, salicylic, stearic, cyclohexylsulfamic, quinic, muconic acid and the like acids; or (2) salts formed when an acidic proton present in the parent compound either (a) is replaced by a metal ion, *e.g.,* an alkali metal ion, an alkaline earth ion or an aluminum ion, or alkali metal or alkaline earth metal hydroxides, such as sodium, potassium, calcium, magnesium, aluminum, lithium, zinc, and barium hydroxide, ammonia, or (b) coordinates with an organic base, such as aliphatic, alicyclic, or aromatic organic amines, such as ammonia, methylamine, dimethylamine, diethylamine, picoline, ethanolamine, diethanolamine, triethanolamine, ethylenediamine, lysine, arginine, ornithine, choline, N,N'-dibenzylethylene-diamine, chloroprocaine, diethanolamine, procaine, N-benzylphenethylamine, N-methylglucamine piperazine, tris(hydroxymethyl)-aminomethane, tetramethylammonium hydroxide, and the like.

Pharmaceutically acceptable salts further include, by way of example only and without limitation, sodium, potassium, calcium, magnesium, ammonium, tetraalkylammonium, and the like, and when the compound contains a basic functionality, salts of non-toxic organic or inorganic acids, such as hydrohalides, e.g. hydrochloride and hydrobromide, sulfate, phosphate, sulfamate, nitrate, acetate, trifluoroacetate, trichloroacetate, propionate, hexanoate, cyclopentylpropionate, glycolate, glutarate, pyruvate, lactate, malonate, succinate, sorbate, ascorbate, malate, maleate, fumarate, tartarate, citrate, benzoate, 3-(4-hydroxybenzoyl)benzoate, picrate, cinnamate, mandelate, phthalate, laurate, methanesulfonate (mesylate), ethanesulfonate, 1,2-ethane-disulfonate, 2-hydroxyethanesulfonate, benzenesulfonate (besylate), 4-chlorobenzenesulfonate, 2-naphthalenesulfonate, 4-toluenesulfonate, camphorate, camphorsulfonate, 4-methylbicyclo[2.2.2]-oct-2-ene-1-carboxylate, glucoheptonate, 3-phenylpropionate, trimethylacetate, tert-butylacetate, lauryl sulfate, gluconate, benzoate, glutamate, hydroxynaphthoate, salicylate, stearate, cyclohexylsulfamate, quinate, muconate, and the like.

The term "amino acid" refers to naturally occurring and synthetic α, β, γ, or δ amino acids, and includes but is not limited to, amino acids found in proteins, *i.e.* glycine, alanine, valine, leucine, isoleucine, methionine, phenylalanine, tryptophan, proline, serine, threonine, cysteine, tyrosine, asparagine, glutamine, aspartate, glutamate, lysine, arginine and histidine. In one embodiment, the amino acid is in the L-configuration. Alternatively, the amino acid can be a derivative of alanyl, valinyl, leucinyl, isoleuccinyl, prolinyl, phenylalaninyl, tryptophanyl, methioninyl, glycinyl, serinyl, threoninyl, cysteinyl, tyrosinyl, asparaginyl, glutaminyl, aspartoyl, glutaroyl, lysinyl, argininyl, histidinyl, β-alanyl, β-valinyl, β-leucinyl, β-isoleuccinyl, β-prolinyl, β-phenylalaninyl, β-tryptophanyl, β-methioninyl, β-glycinyl, β-serinyl, β-threoninyl, β-cysteinyl, β-tyrosinyl, β-asparaginyl, β-glutaminyl, β-aspartoyl, β-glutaroyl, β-lysinyl, β-argininyl, or β-histidinyl.

### Methods of Treatment and Pharmaceutical Preparations and Compositions

VMAT2 inhibitors (and physiologically acceptable salts thereof) may reduce the supply of monoamines in the central nervous system by inhibiting the vesicular monoamine transporter isoform 2 (VMAT2). VMAT2 inhibition results in modulation of the neurotransmitter systems (e.g., dopamine and serotonin).

In one embodiment described herein is the use of a VMAT2 inhibitor for treating schizophrenia or schizoaffective disorder. In another embodiment, the VMAT2 inhibitor comprises (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester, or an isotopic variant thereof; or a pharmaceutically acceptable salt, or polymorph thereof. In some embodiments, provided herein is the use of (*S*)-(2*R*,3*R*,11b*R*)-3-isobutyl-9,10-dimethoxy-2,3,4,6,11b-hexahydro-1H-pyrido[2,1-a]isoquinolin-2-yl 2-amino-3-methylbutanoate di(4-methylbenzenesulfonate), or an isotopic variant thereof, or polymorph thereof for treating schizophrenia or schizoaffective disorder. In certain embodiments, the VMAT2 inhibitor is tetrabenazine (3-isobutyl-9,10-dimethoxy-1,3,4,6,7,11b-hexahydro-2H-pyrido[2,1-a]isoquinolin-2-one). In other embodiments, the VMAT2 inhibitor is deuterated. In other embodiments, the VMAT2 inhibitor is deuterated tetrabenazine (TBZ). Deuterated tetrabenazine includes 3-isobutyl-9,10-d₆-dimethoxy-1,3,4,6,7,11b-hexahydro-2H-pyrido[2,1-a]isoquinolin-2-one (d₆-TBZ). In some embodiments, the VMAT2 inhibitor is deuterated (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester. In certain embodiments, the VMAT2 inhibitor is deuterated (*S*)-(2*R*,3*R*,11b*R*)-3-isobutyl-9,10-dimethoxy-2,3,4,6,7,11b-hexahydro-1H-pyrido[2,l-a]isoquinolin-2-yl 2-amino-3-methylbutanoate di(4-methylbenzenesulfonate). In other embodiments, the VMAT2 inhibitor is (+)α-3-isobutyl-9,10-dimethoxy-1,3,4,6,7,11b-hexahydro-2H-pyrido[2,l-a]isoquinolin-2-ol ((+)α-HTBZ); or (+)α-3-isobutyl-9,10-d₆-dimethoxy-1,3,4,6,7,11b-hexahydro-2H-pyrido[2,1-a]isoquinolin-2-ol (deuterated (+)α-HTBZ). In other embodiments, the VMAT2 inhibitor is (+)β-3-isobutyl-9,10-dimethoxy-1,3,4,6,7,1 1b-hexahydro-2H-pyrido[2,1-a]isoquinolin-2-ol ((+)β-HTBZ); or (+)β-3-isobutyl-9,10-d₆-dimethoxy-1,3,4,6,7,11b-hexahydro-2H-pyrido[2,1-a]isoquinolin-2-ol (deuterated (+)β-HTBZ).

In some embodiments, schizophrenia or schizoaffective disorder includes, but is not limited to, disorders associated with positive and negative symptoms.

In some embodiments, provided herein is a method for the treatment, prevention, or amelioration of one or more symptoms of schizophrenia or schizoaffective disorder, comprising administering to a subject a VMAT2 inhibitor or a pharmaceutical composition comprising a VMAT2 inhibitor described herein. In certain embodiments, the VMAT2 inhibitor comprises (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester, or an isotopic variant thereof; or a pharmaceutically acceptable salt, or polymorph thereof.

In some embodiments, provided herein is a method for the treatment, prevention, or amelioration of one or more symptoms of schizophrenia or schizoaffective disorder, comprising administering to a subject (*S*)-2-amino-3-methyl-butyric acid (2R,3R,11bR)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt or polymorph thereof; or the pharmaceutical compositions described herein. In some embodiments, the VMAT2 inhibitor is (*S*)-(2*R*,3*R*,11b*R*)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate). In certain embodiments, the VMAT2 inhibitor is tetrabenazine (3-isobutyl-9,10-dimethoxy-1,3,4,6,7,11b-hexahydro-2H-pyrido[2,1-a]isoquinolin-2-one). In other embodiments, the VMAT2 inhibitor is deuterated. In some embodiments, the VMAT2 inhibitor is deuterated tetrabenazine (TBZ). Deuterated tetrabenazine includes 3-isobutyl-9,10-d₆-dimethoxy-1,3,4,6,7,11b-hexahydro-2H-pyrido[2,1-a]isoquinolin-2-one (d₆-TBZ). In some embodiments, the VMAT2 inhibitor is deuterated (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester. In certain embodiments, the VMAT2 inhibitor is deuterated (*S*)-(2R,3R,11b*R*)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate). In other embodiments, the VMAT2 inhibitor is (+)α-3-isobutyl-9,10-dimethoxy-1,3,4,6,7,11b-hexahydro-2H-pyrido[2,1-a]isoquinolin-2-ol ((+)α-HTBZ); or (+)α-3-isobutyl-9,10-d₆-dimethoxy-1,3,4,6,7,11b-hexahydro-2H-pyrido[2,1-a]isoquinolin-2-ol (deuterated (+)α-HTBZ). In other embodiments, the VMAT2 inhibitor is (+)β-3-isobutyl-9,10-dimethoxy-1,3,4,6,7,11b-hexahydro-2H-pyrido[2,1-a]isoquinolin-2-ol ((+)β-HTBZ); or (+)β-3-isobutyl-9,10-d₆-dimethoxy-1,3,4,6,7,11b-hexahydro-2H-pyrido[2,1-a]isoquinolin-2-ol (deuterated (+)β-HTBZ).

In other embodiments, (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester, or an isotopic variant thereof; or a pharmaceutically acceptable salt, or polymorph thereof, may prevent, reduce likelihood of occurrence of, slow progression of, delay manifestation of, or treat a symptom associated with schizophrenia or schizoaffective disorder. In some embodiments, the VMAT2 inhibitor is (*S*)-(2*R*,3*R*,11b*R*)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate). In certain embodiments, the VMAT2 inhibitor is tetrabenazine (3-isobutyl-9,10-dimethoxy-1,3,4,6,7,11b-hexahydro-2H-pyrido[2,1-a]isoquinolin-2-one). In other embodiments, the VMAT2 inhibitor is deuterated. In some embodiments, the VMAT2 inhibitor is deuterated tetrabenazine (TBZ). Deuterated tetrabenazine includes 3-isobutyl-9,10-d₆-dimethoxy-1,3,4,6,7,11b-hexahydro-2H-pyrido[2,1-a]isoquinolin-2-one (d₆-TBZ). In some embodiments, the VMAT2 inhibitor is deuterated (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester. In certain embodiments, the VMAT2 inhibitor is deuterated (*S*)-(2*R*,3*R*,11b*R*)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate). In other embodiments, the VMAT2 inhibitor is (+)α-3-isobutyl-9,10-dimethoxy-1,3,4,6,7,11b-hexahydro-2H-pyrido[2,l-a]isoquinolin-2-ol ((+)α-HTBZ); or (+)α-3-isobutyl-9,10-d₆-dimethoxy-1,3,4,6,7,1 1b-hexahydro-2H-pyrido[2,1-a]isoquinolin-2-ol (deuterated 3(+)α-HTBZ). In other embodiments, the VMAT2 inhibitor is (+)β-3-isobutyl-9,10-dimethoxy-1,3,4,6,7,1 1b-hexahydro-2H-pyrido[2,1-a]isoquinolin-2-ol ((+)β-HTBZ); or (+)β-3-isobutyl-9,10-d₆-dimethoxy-1,3,4,6,7,1 1b-hexahydro-2H-pyrido[2,1-a]isoquinolin-2-ol (deuterated (+)β-HTBZ).

In some embodiments, in a subject with schizophrenia or schizoaffective disorder, treatment with (*S*)-2-amino-3-methyl-butyric acid (2*R*,3*R*,11b*R*)-3-isobutyl-9,10-dimethoxy-1,3,4,6,7,1 1b-hexahydro-2H-pyrido[2,l-a]isoquinolin-2-yl ester, or an isotopic variant thereof, or a pharmaceutically acceptable salt, or polymorph thereof may improve or effectively reduce one or more symptoms associated with schizophrenia or schizoaffective disorder. In some embodiments, the symptoms include, but are not limied to, positive symptoms and negative symptoms. In some embodiments, the symptoms include, by way of example, positive symptoms such as delusions, disordered thoughts and speech, and tactile, auditory, visual, olfactory and gustatory hallucinations, typically regarded as manifestations of psychosis. Hallucinations are also typically related to the content of the delusional theme. In some embodiments, the symptoms include, by way of example, negative symptoms such as flat expressions or little emotion, poverty of speech, inability to experience pleasure, lack of desire to form relationships, and lack of motivation. In some embodiments, the negative symptoms include those that appear to contribute to poor quality of life, functional ability, and the burden on others.

In some embodiments, in a subject with schizophrenia or schizoaffective disorder, treatment with (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof may manage behavioral problems associated with schizophrenia or schizoaffective disorder, including but not limited to those associated with positive and negative (or deficit) symptoms.

The causes of schizophrenia or schizoaffective disorder include environmental and genetic factors. Possible environmental factors include being raised in a city, cannabis use, certain infections, parental age, and poor nutrition during pregnancy. Genetic factors include a variety of common and rare genetic variants. Diagnosis is based on observed behavior, the person's reported experiences, and reports of others familiar with the person. During diagnosis a person's culture must also be taken into account. Schizophrenia does not imply a "split personality" or "multiple personality disorder" - conditions with which it is often confused in public perception.

The mainstay of treatment is antipsychotic medication along with counselling, job training, and social rehabilitation. It is unclear if typical or atypical antipsychotics are better. In more serious situtations-where there is risk to self or others-involuntary hospitalization may be necessary, although hospital stays are now shorter and less frequent than they once were.

Psychotic symptoms may be present in several other mental disorders, including bipolar disorder, borderline personality disorder, drug intoxication and drug-induced psychosis. Delusions ("non-bizarre") are also present in delusional disorder, and social withdrawal in social anxiety disorder, avoidant personality disorder and schizotypal personality disorder. Schizotypal personality disorder has symptoms that are similar but less severe than those of schizophrenia. Schizophrenia occurs along with obsessive-compulsive disorder (OCD) considerably more often than could be explained by chance, although it can be difficult to distinguish obsessions that occur in OCD from the delusions of schizophrenia. A few people withdrawing from benzodiazepines experience a severe withdrawal syndrome which may last a long time. It can resemble schizophrenia and be misdiagnosed as such.

A more general medical and neurological examination may be needed to rule out medical illnesses which may rarely produce psychotic schizophrenia-like symptoms, such as metabolic disturbance, systemic infection, syphilis, HIV/AIDS, epilepsy, limbic encephalitis, and brain lesions. Stroke, multiple sclerosis, hyperthyroidism, hypothyroidism and dementias such as Alzheimer's disease, Huntington's disease, frontotemporal dementia and Lewy Body dementia may also be associated with schizophrenia-like psychotic symptoms. It may be necessary to rule out a delirium, which can be distinguished by visual hallucinations, acute onset and fluctuating level of consciousness, and indicates an underlying medical illness. In children hallucinations must be separated from typical childhood fantasies.

Schizophrenia is associated with subtle differences in brain structures, found in forty to fifty percent of cases, and in brain chemistry during acute psychotic states. Studies using neuropsychological tests and brain imaging technologies such as fMRI (functional magnetic resonance imaging) and PET to examine functional differences in brain activity have shown that differences seem to occur most commonly in the frontal lobes, hippocampus and temporal lobes. Reductions in brain volume, smaller than those found in Alzheimer's disease, have been reported in areas of the frontal cortex and temporal lobes. It is uncertain whether these volumetric changes are progressive or exist prior to the onset of the disease. These differences have been linked to the neurocognitive deficits often associated with schizophrenia.

Schizophrenia is diagnosed based on criteria in either the American Psychiatric Association's fifth edition of the Diagnostic and Statistical Manual of Mental Disorders (DSM 5), or the World Health Organization's International Statistical Classification of Diseases and Related Health Problems (ICD-10). These criteria use the self-reported experiences of the person and reported abnormalities in behavior, followed by a clinical assessment by a mental health professional. Symptoms associated with schizophrenia occur along a continuum in the population and must reach a certain severity before a diagnosis is made. The Positive and Negative Syndrome Scale (PANSS) is a 30-item, 7-point rating instrument used in measuring the severity of symptoms in subjects with schizophrenia. The assessment is administered over an approximately 45 minute period by a trained interviewer. Each item on the PANSS is accompanied by a complete definition as well as detailed anchoring criteria for all the seven rating points (Stanley R. Kay, The Positive and Negative Syndrome Scale (PANSS) for Schizophrenia, Scizophrenia Bulletin, 1987, 13, 262-276). The 30 items are divided into three subscales - Positive Symptoms (delusions and hallucinations), Negative Symptoms (social withdrawal, flattened/blunted affect), and General Psychopathology. These 3 subscales may be summed to give a Total score.

In certain embodiments, in a subject with schizophrenia or schizoaffective disorder, treatment with (*S*)-2-amino-3-methyl-butyric acid (2*R*,3*R*,11b*R*)-3-isobutyl-9,10-dimethoxy-1,3,4,6,7, 1 1b-hexahydro-2H-pyrido[2,l-a]isoquinolin-2-yl ester, or an isotopic variant thereof, or a pharmaceutically acceptable salt, or polymorph thereof may improve or effectively reduce one or more symptoms associated with schizophrenia or schizoaffective disorder. In some embodiments, the VMAT2 inhibitor is (*S*)-(2*R*,3*R*,11b*R*)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate). In certain embodiments, the VMAT2 inhibitor is tetrabenazine (3-isobutyl-9,10-dimethoxy-1,3,4,6,7,11b-hexahydro-2H-pyrido[2,1-a]isoquinolin-2-one). In other embodiments, the VMAT2 inhibitor is deuterated. In some embodiments, the VMAT2 inhibitor is deuterated tetrabenazine (TBZ). Deuterated tetrabenazine includes 3-isobutyl-9,10-d₆-dimethoxy-1,3,4,6,7,11b-hexahydro-2H-pyrido[2,1-a]isoquinolin-2-one (d₆-TBZ). In some embodiments, the VMAT2 inhibitor is deuterated (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester. In certain embodiments, the VMAT2 inhibitor is deuterated (*S*)-(2*R*,3*R*,11b*R*)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate). In other embodiments, the VMAT2 inhibitor is (+)α-3-isobutyl-9,10-dimethoxy-1,3,4,6,7,11b-hexahydro-2H-pyrido[2,l-a]isoquinolin-2-ol ((+)α-HTBZ); or (+)α-3-isobutyl-9,10-d₆-dimethoxy-1,3,4,6,7,1 1b-hexahydro-2H-pyrido[2,1-a]isoquinolin-2-ol (deuterated (+)α-HTBZ). In other embodiments, the VMAT2 inhibitor is (+)β-3-isobutyl-9,10-dimethoxy-1,3,4,6,7,1 1b-hexahydro-2H-pyrido[2,1-a]isoquinolin-2-ol ((+)β-HTBZ); or (+)β-3-isobutyl-9,10-d₆-dimethoxy-1,3,4,6,7,1 1b-hexahydro-2H-pyrido[2,1-a]isoquinolin-2-ol (deuterated (+)β-HTBZ).

In some embodiments, the subject includes a subject who has been diagnosed by a person skilled in the medical art. Symptoms associated with schizophrenia or schizoaffective disorder include, but are not limited to, positive symptoms and negative symptoms. In some embodiments, individuals with schizophrenia may experience hallucinations (most reported are hearing voices), delusions (often bizarre or persecutory in nature), and disorganized thinking and speech. The last may range from loss of train of thought, to sentences only loosely connected in meaning, to speech that is not understandable known as word salad. Social withdrawal, sloppiness of dress and hygiene, and loss of motivation and judgment are all common in schizophrenia. Distortions of self-experience such as feeling as if one's thoughts or feelings are not really one's own to believing thoughts are being inserted into one's mind, sometimes termed passivity phenomena, are also common. There is often an observable pattern of emotional difficulty, for example lack of responsiveness. Impairment in social cognition is associated with schizophrenia, as are symptoms of paranoia. Social isolation commonly occurs. Difficulties in working and long-term memory, attention, executive functioning, and speed of processing also commonly occur. In one uncommon subtype, the person may be largely mute, remain motionless in bizarre postures, or exhibit purposeless agitation, all signs of catatonia. About 30 to 50 percent of people with schizophrenia fail to accept that they have an illness or comply with their recommended treatment. Treatment may have some effect on insight. People with schizophrenia often find facial emotion perception to be difficult.

Valbenazine can be prepared according to U.S. Patent Nos. 8,039,627 and 8,357,697, the disclosure of each of which is incorporated herein by reference in its entirety. Tetrabenazine may be administered by a variety of methods including the formulations disclosed in PCT Publications WO 2010/018408, WO 2011/019956, and WO 2014/047167, the disclosure of each of which is incorporated herein by reference in its entirety. In another embodiment, the valbenazine for use in the compositions and methods provided herein is in polymorphic Form I as disclosed in U.S. Serial No. 15/338,214, the disclosure of which is incorporated herein by reference in its entirety.

In another embodiment, d₆-tetrabenazine as disclosed in U.S. Patent No. 8,524,733 is administered resulting in an appropriate concentration over a specified period of time of metabolite (+)α-3-isobutyl-9,10-d₆-dimethoxy-1,3,4,6,7,11bhexahydro-2H-pyrido[2,1-a]isoquinolin-2-ol (deuterated (+)α-HTBZ) or deuterated (+)β-HTBZ in the plasma). The d₆-tetrabenazine may be administered by a variety of methods including the formulations as disclosed in PCT Publication WO 2014/047167, the disclosure of which is incorporated herein by reference in its entirety.

### Pharmaceutical compositions

Also provided herein is a pharmaceutical composition for use in treating schizophrenia or schizoaffective disorder, comprising (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester, or an isotopic variant thereof; or a pharmaceutically acceptable salt, or polymorph thereof, as an active pharmaceutical ingredient, in combination with one or more pharmaceutically acceptable carriers or excipients. In some embodiments, the pharmaceutical composition comprises (*S*)-(2*R*,3*R*,11b*R*)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate). In certain embodiments, the pharmaceutical composition comprises a VMAT2 inhibitor. In certain embodiments, the VMAT2 inhibitor is tetrabenazine (3-isobutyl-9,10-dimethoxy-1,3,4,6,7,1 1b-hexahydro-2H-pyrido[2,1-a]isoquinolin-2-one). In other embodiments, the pharmaceutical composition comprises a deuterated VMAT2 inhibitor. In some embodiments, the VMAT2 inhibitor is deuterated tetrabenazine (TBZ). Deuterated tetrabenazine includes 3-isobutyl-9,10-d₆-dimethoxy-1,3,4,6,7,11b-hexahydro-2H-pyrido[2,1-a]isoquinolin-2-one (d₆-TBZ). In some embodiments, the VMAT2 inhibitor is deuterated (*S*)-2-amino-3-methyl-butyric acid (2*R*,3*R*,11bR)-3-isobutyl-9, 10-dimethoxy-1,3,4,6,7,1 1b-hexahydro-2H-pyrido[2,l-a]isoquinolin-2-yl ester. In certain embodiments, the VMAT2 inhibitor is deuterated (*S*)-(2*R*,3*R*,11b*R*)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate). In other embodiments, the VMAT2 inhibitor is (+)α-3-isobutyl-9,10-dimethoxy-1,3,4,6,7,11b-hexahydro-2H-pyrido[2,1-a]isoquinolin-2-ol ((+)α-HTBZ); or (+)α-3-isobutyl-9,10-d₆-dimethoxy-1,3,4,6,7,11b-hexahydro-2H-pyrido[2,1-a]isoquinolin-2-ol (deuterated (+)α-HTBZ). In other embodiments, the VMAT2 inhibitor is (+)β-3-isobutyl-9,10-dimethoxy-1,3,4,6,7,11b-hexahydro-2H-pyrido[2,1-a]isoquinolin-2-ol ((+)β-HTBZ); or (+)β-3-isobutyl-9,10-d₆-dimethoxy-1,3,4,6,7,11b-hexahydro-2H-pyrido[2,1-a]isoquinolin-2-ol (deuterated (+)β-HTBZ).

The choice of excipient, to a large extent, depends on factors, such as the particular mode of administration, the effect of the excipient on the solubility and stability of the active ingredient, and the nature of the dosage form.

The pharmaceutical compositions provided herein may be provided in unit dosage forms or multiple-dosage forms. Unit-dosage forms, as used herein, refer to physically discrete units suitable for administration to human and animal subjects and packaged individually as is known in the art. Each unit-dose contains a predetermined quantity of the active ingredient(s) sufficient to produce the desired therapeutic effect, in association with the required pharmaceutical carriers or excipients. Examples of unit-dosage forms include ampouls, syringes, and individually packaged tablets and capsules. Unit dosage forms may be administered in fractions or multiples thereof. A multiple-dosage form is a plurality of identical unit-dosage forms packaged in a single container to be administered in segregated unit-dosage form. Examples of multiple-dosage forms include vials, bottles of tablets or capsules, or bottles of pints or gallons.

The pharmaceutical compositions provided herein may be administered alone, or in combination with one or more other compounds provided herein, one or more other active ingredients. The pharmaceutical compositions provided herein may be formulated in various dosage forms for oral, parenteral, and topical administration. The pharmaceutical compositions may also be formulated as a modified release dosage form, including delayed-, extended-, prolonged-, sustained-, pulsatile-, controlled-, accelerated- and fast-, targeted-, programmed-release, and gastric retention dosage forms. These dosage forms can be prepared according to conventional methods and techniques known to those skilled in the art (*see,* Remington: The Science and Practice of Pharmacy*,* supra; Modified-Release Drug Delivery Technology, Rathbone et al., Eds., Drugs and the Pharmaceutical Science, Marcel Dekker, Inc.: New York, NY, 2002; Vol. 126).

The pharmaceutical compositions provided herein may be administered at once, or multiple times at intervals of time. It is understood that the precise dosage and duration of treatment may vary with the age, weight, and condition of the patient being treated, and may be determined empirically using known testing protocols or by extrapolation from *in vivo* or *in vitro* test or diagnostic data. It is further understood that for any particular individual, specific dosage regimens should be adjusted over time according to the individual need and the professional judgment of the person administering or supervising the administration of the formulations.

### Oral Administration

The pharmaceutical compositions provided herein may be provided in solid, semisolid, or liquid dosage forms for oral administration. As used herein, oral administration also include buccal, lingual, and sublingual administration. Suitable oral dosage forms include, but are not limited to, tablets, capsules, pills, troches, lozenges, pastilles, cachets, pellets, medicated chewing gum, granules, bulk powders, effervescent or non-effervescent powders or granules, solutions, emulsions, suspensions, solutions, wafers, sprinkles, elixirs, and syrups. In addition to the active ingredient(s), the pharmaceutical compositions may contain one or more pharmaceutically acceptable carriers or excipients, including, but not limited to, binders, fillers, diluents, disintegrants, wetting agents, lubricants, glidants, coloring agents, dye-migration inhibitors, sweetening agents, and flavoring agents.

Binders or granulators impart cohesiveness to a tablet to ensure the tablet remaining intact after compression. Suitable binders or granulators include, but are not limited to, starches, such as corn starch, potato starch, and pre-gelatinized starch (e.g., STARCH 1500); gelatin; sugars, such as sucrose, glucose, dextrose, molasses, and lactose; natural and synthetic gums, such as acacia, alginic acid, alginates, extract of Irish moss, Panwar gum, ghatti gum, mucilage of isabgol husks, carboxymethylcellulose, methylcellulose, polyvinylpyrrolidone (PVP), Veegum, larch arabogalactan, powdered tragacanth, and guar gum; celluloses, such as ethyl cellulose, cellulose acetate, carboxymethyl cellulose calcium, sodium carboxymethyl cellulose, methyl cellulose, hydroxyethylcellulose (HEC), hydroxypropylcellulose (HPC), hydroxypropyl methyl cellulose (HPMC); microcrystalline celluloses, such as AVICEL-PH-101, AVICEL-PH-103, AVICEL RC-581, AVICEL-PH-105 (FMC Corp., Marcus Hook, PA); and mixtures thereof. Suitable fillers include, but are not limited to, talc, calcium carbonate, microcrystalline cellulose, powdered cellulose, dextrates, kaolin, mannitol, silicic acid, sorbitol, starch, pregelatinized starch, and mixtures thereof. The binder or filler may be present from about 50 to about 99% by weight in the pharmaceutical compositions provided herein.

Suitable diluents include, but are not limited to, dicalcium phosphate, calcium sulfate, lactose, sorbitol, sucrose, inositol, cellulose, kaolin, mannitol, sodium chloride, dry starch, and powdered sugar. Certain diluents, such as mannitol, lactose, sorbitol, sucrose, and inositol, when present in sufficient quantity, can impart properties to some compressed tablets that permit disintegration in the mouth by chewing. Such compressed tablets can be used as chewable tablets.

Suitable disintegrants include, but are not limited to, agar; bentonite; celluloses, such as methylcellulose and carboxymethylcellulose; wood products; natural sponge; cation-exchange resins; alginic acid; gums, such as guar gum and Vee gum HV; citrus pulp; cross-linked celluloses, such as croscarmellose; cross-linked polymers, such as crospovidone; cross-linked starches; calcium carbonate; microcrystalline cellulose, such as sodium starch glycolate; polacrilin potassium; starches, such as com starch, potato starch, tapioca starch, and pre-gelatinized starch; clays; aligns; and mixtures thereof. The amount of disintegrant in the pharmaceutical compositions provided herein varies upon the type of formulation, and is readily discernible to those of ordinary skill in the art. The pharmaceutical compositions provided herein may contain from about 0.5 to about 15% or from about 1 to about 5% by weight of a disintegrant.

Suitable lubricants include, but are not limited to, calcium stearate; magnesium stearate; mineral oil; light mineral oil; glycerin; sorbitol; mannitol; glycols, such as glycerol behenate and polyethylene glycol (PEG); stearic acid; sodium lauryl sulfate; talc; hydrogenated vegetable oil, including peanut oil, cottonseed oil, sunflower oil, sesame oil, olive oil, com oil, and soybean oil; zinc stearate; ethyl oleate; ethyl laureate; agar; starch; lycopodium; silica or silica gels, such as AEROSIL^{®} 200 (W.R. Grace Co., Baltimore, MD) and CAB-0-SIL^{®} (Cabot Co. of Boston, MA); and mixtures thereof. The pharmaceutical compositions provided herein may contain about 0.1 to about 5% by weight of a lubricant.

Suitable glidants include colloidal silicon dioxide, CAB-0-SIL^{®} (Cabot Co. of Boston, MA), and asbestos-free talc. Coloring agents include any of the approved, certified, water soluble FD&C dyes, and water insoluble FD&C dyes suspended on alumina hydrate, and color lakes and mixtures thereof. A color lake is the combination by adsorption of a water-soluble dye to a hydrous oxide of a heavy metal, resulting in an insoluble form of the dye. Flavoring agents include natural flavors extracted from plants, such as fruits, and synthetic blends of compounds which produce a pleasant taste sensation, such as peppermint and methyl salicylate. Sweetening agents include sucrose, lactose, mannitol, syrups, glycerin, and artificial sweeteners, such as saccharin and aspartame. Suitable emulsifying agents include gelatin, acacia, tragacanth, bentonite, and surfactants, such as polyoxyethylene sorbitan monooleate (TWEEN^{®} 20), polyoxyethylene sorbitan monooleate 80 (TWEEN^{®} 80), and triethanolamine oleate. Suspending and dispersing agents include sodium carboxymethylcellulose, pectin, tragacanth, Veegum, acacia, sodium carbomethylcellulose, hydroxypropyl methylcellulose, and polyvinylpyrolidone. Preservatives include glycerin, methyl and propylparaben, benzoic add, sodium benzoate and alcohol. Wetting agents include propylene glycol monostearate, sorbitan monooleate, diethylene glycol monolaurate, and polyoxyethylene lauryl ether. Solvents include glycerin, sorbitol, ethyl alcohol, and syrup. Examples of non-aqueous liquids utilized in emulsions include mineral oil and cottonseed oil. Organic acids include citric and tartaric acid. Sources of carbon dioxide include sodium bicarbonate and sodium carbonate.

It should be understood that many carriers and excipients may serve several functions, even within the same formulation. The pharmaceutical compositions provided herein may be provided as compressed tablets, tablet triturates, chewable lozenges, rapidly dissolving tablets, multiple compressed tablets, or enteric-coating tablets, sugar-coated, or film-coated tablets. Enteric coated tablets are compressed tablets coated with substances that resist the action of stomach acid but dissolve or disintegrate in the intestine, thus protecting the active ingredients from the acidic environment of the stomach. Enteric-coatings include, but are not limited to, fatty acids, fats, phenylsalicylate, waxes, shellac, ammoniated shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms may be prepared from the active ingredient in powdered, crystalline, or granular forms, alone or in combination with one or more carriers or excipients described herein, including binders, disintegrants, controlled-release polymers, lubricants, diluents, and/or colorants. Flavoring and sweetening agents are especially useful in the formation of chewable tablets and lozenges.

The pharmaceutical compositions provided herein may be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and propyl-parabens, and sorbic acid. The liquid, semisolid, and solid dosage forms provided herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient.

The pharmaceutical compositions provided herein may be provided in liquid and semisolid dosage forms, including emulsions, solutions, suspensions, elixirs, and syrups. An emulsion is a two-phase system, in which one liquid is dispersed in the form of small globules throughout another liquid, which can be oil-in-water or water-in-oil. Emulsions may include a pharmaceutically acceptable non-aqueous liquids or solvent, emulsifying agent, and preservative. Suspensions may include a pharmaceutically acceptable suspending agent and preservative. Aqueous alcoholic solutions may include a pharmaceutically acceptable acetal, such as a di(lower alkyl) acetal of a lower alkyl aldehyde (the term "lower" means an alkyl having between 1 and 6 carbon atoms), *e.g*., acetaldehyde diethyl acetal; and a water-miscible solvent having one or more hydroxyl groups, such as propylene glycol and ethanol. Elixirs are clear, sweetened, and hydroalcoholic solutions. Syrups are concentrated aqueous solutions of a sugar, for example, sucrose, and may also contain a preservative. For a liquid dosage form, for example, a solution in a polyethylene glycol may be diluted with a sufficient quantity of a pharmaceutically acceptable liquid carrier, *e.g.*, water, to be measured conveniently for administration.

Other useful liquid and semisolid dosage forms include, but are not limited to, those containing the active ingredient(s) provided herein, and a dialkylated mono- or polyalkylene glycol, including, 1,2-dimethoxymethane, diglyme, triglyme, tetraglyme, polyethylene glycol-350-dimethyl ether, polyethylene glycol-550-dimethyl ether, polyethylene glycol-750-dimethyl ether, wherein 350, 550, and 750 refer to the approximate average molecular weight of the polyethylene glycol. These formulations may further comprise one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, bisulfite, sodium metabisulfite, thiodipropionic acid and its esters, and dithiocarbamates.

The pharmaceutical compositions provided herein for oral administration may be also provided in the forms of liposomes, micelles, microspheres, or nanosystems. Micellar dosage forms can be prepared as described in U.S. Pat. No. 6,350,458.

The pharmaceutical compositions provided herein may be provided as noneffervescent or effervescent, granules and powders, to be reconstituted into a liquid dosage form. Pharmaceutically acceptable carriers and excipients used in the non-effervescent granules or powders may include diluents, sweeteners, and wetting agents. Pharmaceutically acceptable carriers and excipients used in the effervescent granules or powders may include organic acids and a source of carbon dioxide.

Coloring and flavoring agents can be used in all of the above dosage forms. The pharmaceutical compositions provided herein may be formulated as immediate or modified release dosage forms, including delayed-, sustained, pulsed-, controlled, targeted-, and programmed-release forms.

The pharmaceutical compositions provided herein may be co-formulated with other active ingredients which do not impair the desired therapeutic action, or with substances that supplement the desired action, such as antacids, proton pump inhibitors, and H₂-receptor antagonists.

The pharmaceutical compositions provided herein may be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, and subcutaneous administration.

### Parenteral Administration

The pharmaceutical compositions provided herein may be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science *(see,* Remington: The Science and Practice of Pharmacy*,* supra).

The pharmaceutical compositions intended for parenteral administration may include one or more pharmaceutically acceptable carriers and excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents, and inert gases.

Suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Non-aqueous vehicles include, but are not limited to, fixed oils of vegetable origin, castor oil, com oil, cottonseed oil, olive oil, peanut oil, peppermint oil, safflower oil, sesame oil, soybean oil, hydrogenated vegetable oils, hydrogenated soybean oil, and medium-chain triglycerides of coconut oil, and palm seed oil. Water-miscible vehicles include, but are not limited to, ethanol, 1 ,3-butanediol, liquid polyethylene glycol (e.g., polyethylene glycol 300 and polyethylene glycol 400), propylene glycol, glycerin, N-methyl-2-pyrrolidone, dimethylacetamide, and dimethylsulfoxide.

Suitable antimicrobial agents or preservatives include, but are not limited to, phenols, cresols, mercurials, benzyl alcohol, chlorobutanol, methyl and propyl phydroxybenzates, thimerosal, benzalkonium chloride, benzethonium chloride, methyl- and propyl-parabens, and sorbic acid. Suitable isotonic agents include, but are not limited to, sodium chloride, glycerin, and dextrose. Suitable buffering agents include, but are not limited to, phosphate and citrate. Suitable antioxidants are those as described herein, including bisulfite and sodium metabisulfite. Suitable local anesthetics include, but are not limited to, procaine hydrochloride. Suitable suspending and dispersing agents are those as described herein, including sodium carboxymethylcelluose, hydroxypropyl methylcellulose, and polyvinylpyrrolidone. Suitable emulsifying agents include those described herein, including polyoxyethylene sorbitan monolaurate, polyoxyethylene sorbitan monooleate 80, and triethanolamine oleate. Suitable sequestering or chelating agents include, but are not limited to EDTA. Suitable pH adjusting agents include, but are not limited to, sodium hydroxide, hydrochloric acid, citric acid, and lactic acid. Suitable complexing agents include, but are not limited to, cyclodextrins, including alpha-cyclodextrin, beta-cyclodextrin, hydroxypropyl-beta-cyclodextrin, sulfobutylether-beta-cyclodextrin, and sulfobutylether 7-beta-cyclodextrin (CAPTISOL^{®}, CyDex, Lenexa, KS).

The pharmaceutical compositions provided herein may be formulated for single or multiple dosage administration. The single dosage formulations are packaged in an ampule, a vial, or a syringe. The multiple dosage parenteral formulations must contain an antimicrobial agent at bacteriostatic or fungistatic concentrations. All parenteral formulations must be sterile, as known and practiced in the art.

In one embodiment, the pharmaceutical compositions are provided as ready-to-use sterile solutions. In another embodiment, the pharmaceutical compositions are provided as sterile dry soluble products, including lyophilized powders and hypodermic tablets, to be reconstituted with a vehicle prior to use. In yet another embodiment, the pharmaceutical compositions are provided as ready-to-use sterile suspensions. In yet another embodiment, the pharmaceutical compositions are provided as sterile dry insoluble products to be reconstituted with a vehicle prior to use. In still another embodiment, the pharmaceutical compositions are provided as ready-to-use sterile emulsions.

The pharmaceutical compositions provided herein may be formulated as immediate or modified release dosage forms, including delayed-, sustained, pulsed-, controlled, targeted-, and programmed-release forms.

The pharmaceutical compositions may be formulated as a suspension, solid, semisolid, or thixotropic liquid, for administration as an implanted depot. In one embodiment, the pharmaceutical compositions provided herein are dispersed in a solid inner matrix, which is surrounded by an outer polymeric membrane that is insoluble in body fluids but allows the active ingredient in the pharmaceutical compositions diffuse through.

Suitable inner matrixes include polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers, such as hydrogels of esters of acrylic and methacrylic acid, collagen, cross-linked polyvinylalcohol, and cross-linked partially hydrolyzed polyvinyl acetate.

Suitable outer polymeric membranes include polyethylene, polypropylene, ethylene/propylene copolymers, ethylene/ethyl acrylate copolymers, ethylene/vinyl acetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer.

### Topical Administration

The pharmaceutical compositions provided herein may be administered topically to the skin, orifices, or mucosa. The topical administration, as used herein, include (intra)dermal, conjuctival, intracorneal, intraocular, ophthalmic, auricular, transdermal, nasal, vaginal, uretheral, respiratory, and rectal administration.

The pharmaceutical compositions provided herein may be formulated in any dosage forms that are suitable for topical administration for local or systemic effect, including emulsions, solutions, suspensions, creams, gels, hydrogels, ointments, dusting powders, dressings, elixirs, lotions, suspensions, tinctures, pastes, foams, films, aerosols, irrigations, sprays, suppositories, bandages, dermal patches. The topical formulation of the pharmaceutical compositions provided herein may also comprise liposomes, micelles, microspheres, nanosystems, and mixtures thereof.

Pharmaceutically acceptable carriers and excipients suitable for use in the topical formulations provided herein include, but are not limited to, aqueous vehicles, water miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, penetration enhancers, cryoprotectants, lyoprotectants, thickening agents, and inert gases.

The pharmaceutical compositions may also be administered topically by electroporation, iontophoresis, phonophoresis, sonophoresis and microneedle or needle-free injection, such as POWDERJECT^{™} (Chiron Corp., Emeryville, CA), and BIOJECT^{™} (Bioject Medical Technologies Inc., Tualatin, OR).

The pharmaceutical compositions provided herein may be provided in the forms of ointments, creams, and gels. Suitable ointment vehicles include oleaginous or hydrocarbon bases, including such as lard, benzoinated lard, olive oil, cottonseed oil, and other oils, white petrolatum; emulsifiable or absorption bases, such as hydrophilic petrolatum, hydroxystearin sulfate, and anhydrous lanolin; water-removable bases, such as hydrophilic ointment; water-soluble ointment bases, including polyethylene glycols of varying molecular weight; emulsion bases, either water-in-oil (W/O) emulsions or oil-in-water (O/W) emulsions, including cetyl alcohol, glyceryl monostearate, lanolin, and stearic acid (*see,* Remington: The Science and Practice of Pharmacy*,* supra). These vehicles are emollient but generally require addition of antioxidants and preservatives.

Suitable cream base can be oil-in-water or water-in-oil. Cream vehicles may be water-washable, and contain an oil phase, an emulsifier, and an aqueous phase. The oil phase is also called the "internal" phase, which is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation may be a nonionic, anionic, cationic, or amphoteric surfactant.

Gels are semisolid, suspension-type systems. Single-phase gels contain organic macromolecules distributed substantially uniformly throughout the liquid carrier. Suitable gelling agents include crosslinked acrylic acid polymers, such as carbomers, carboxypolyalkylenes, Carbopol^{®}; hydrophilic polymers, such as polyethylene oxides, polyoxyethylene-polyoxypropylene copolymers, and polyvinylalcohol; cellulosic polymers, such as hydroxypropyl cellulose, hydroxyethyl cellulose, hydroxypropyl methylcellulose, hydroxypropyl methylcellulose phthalate, and methylcellulose; gums, such as tragacanth and xanthan gum; sodium alginate; and gelatin. In order to prepare a uniform gel, dispersing agents such as alcohol or glycerin can be added, or the gelling agent can be dispersed by trituration, mechanical mixing, and/or stirring.

The pharmaceutical compositions provided herein may be administered rectally, urethrally, vaginally, or perivaginally in the forms of suppositories, pessaries, bougies, poultices or cataplasm, pastes, powders, dressings, creams, plasters, contraceptives, ointments, solutions, emulsions, suspensions, tampons, gels, foams, sprays, or enemas. These dosage forms can be manufactured using conventional processes as described in Remington: *The Science and Practice of Pharmacy,* supra.

Rectal, urethral, and vaginal suppositories are solid bodies for insertion into body orifices, which are solid at ordinary temperatures but melt or soften at body temperature to release the active ingredient(s) inside the orifices. Pharmaceutically acceptable carriers utilized in rectal and vaginal suppositories include vehicles, such as stiffening agents, which produce a melting point in the proximity of body temperature, when formulated with the pharmaceutical compositions provided herein; and antioxidants as described herein, including bisulfite and sodium metabisulfite. Suitable vehicles include, but are not limited to, cocoa butter (theobroma oil), glycerin-gelatin, carbowax (polyoxyethylene glycol), spermaceti, paraffin, white and yellow wax, and appropriate mixtures of mono-, di- and triglycerides of fatty acids, hydrogels, such as polyvinyl alcohol, hydroxyethyl methacrylate, polyacrylic acid; glycerinated gelatin. Combinations of the various vehicles may be used. Rectal and vaginal suppositories may be prepared by the compressed method or molding. The typical weight of a rectal and vaginal suppository is about 2 to 3 g.

The pharmaceutical compositions provided herein may be administered ophthalmically in the forms of solutions, suspensions, ointments, emulsions, gel-forming solutions, powders for solutions, gels, ocular inserts, and implants.

The pharmaceutical compositions provided herein may be administered intranasally or by inhalation to the respiratory tract. The pharmaceutical compositions may be provided in the form of an aerosol or solution for delivery using a pressurized container, pump, spray, atomizer, such as an atomizer using electrohydrodynamics to produce a fine mist, or nebulizer, alone or in combination with a suitable propellant, such as 1,1,1,2-tetrafluoroethane or 1,1,1,2,3,3,3-heptafluoropropane. The pharmaceutical compositions may also be provided as a dry powder for insufflation, alone or in combination with an inert carrier such as lactose or phospholipids; and nasal drops. For intranasal use, the powder may comprise a bioadhesive agent, including chitosan or cyclodextrin.

Solutions or suspensions for use in a pressurized container, pump, spray, atomizer, or nebulizer may be formulated to contain ethanol, aqueous ethanol, or a suitable alternative agent for dispersing, solubilizing, or extending release of the active ingredient provided herein, a propellant as solvent; and/or a surfactant, such as sorbitan trioleate, oleic acid, or an oligolactic acid.

The pharmaceutical compositions provided herein may be micronized to a size suitable for delivery by inhalation, such as 50 micrometers or less, or 10 micrometers or less. Particles of such sizes may be prepared using a comminuting method known to those skilled in the art, such as spiral jet milling, fluid bed jet milling, supercritical fluid processing to form nanoparticles, high pressure homogenization, or spray drying.

Capsules, blisters and cartridges for use in an inhaler or insufflator may be formulated to contain a powder mix of the pharmaceutical compositions provided herein; a suitable powder base, such as lactose or starch; and a performance modifier, such as *l*-leucine, mannitol, or magnesium stearate. The lactose may be anhydrous or in the form of the monohydrate. Other suitable excipients include dextran, glucose, maltose, sorbitol, xylitol, fructose, sucrose, and trehalose. The pharmaceutical compositions provided herein for inhaled/intranasal administration may further comprise a suitable flavor, such as menthol and levomenthol, or sweeteners, such as saccharin or saccharin sodium.

The pharmaceutical compositions provided herein for topical administration may be formulated to be immediate release or modified release, including delayed-, sustained-, pulsed-, controlled-, targeted, and programmed release.

### Modified Release

The pharmaceutical compositions provided herein may be formulated as a modified release dosage form. As used herein, the term "modified release" refers to a dosage form in which the rate or place of release of the active ingredient(s) is different from that of an immediate dosage form when administered by the same route. Modified release dosage forms include delayed-, extended-, prolonged-, sustained-, pulsatile- or pulsed-, controlled-, accelerated- and fast-, targeted-, programmed-release, and gastric retention dosage forms. The pharmaceutical compositions in modified release dosage forms can be prepared using a variety of modified release devices and methods known to those skilled in the art, including, but not limited to, matrix controlled release devices, osmotic controlled release devices, multiparticulate controlled release devices, ion-exchange resins, enteric coatings, multilayered coatings, microspheres, liposomes, and combinations thereof. The release rate of the active ingredient(s) can also be modified by varying the particle sizes and polymorphorism of the active ingredient(s).

Examples of modified release include, but are not limited to, those described in U.S. Pat. Nos.: 3,845,770; 3,916,899; 3,536,809; 3,598,123; 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358; and 6,699,500.

### Matrix Controlled Release Devices

The pharmaceutical compositions provided herein in a modified release dosage form may be fabricated using a matrix controlled release device known to those skilled in the art *(see,* Takada et al. in "Encyclopedia of Controlled Drug Delivery," Vol. 2, Mathiowitz ed., Wiley, 1999).

In one embodiment, the pharmaceutical compositions provided herein in a modified release dosage form is formulated using an erodible matrix device, which is water swellable, erodible, or soluble polymers, including synthetic polymers, and naturally occurring polymers and derivatives, such as polysaccharides and proteins.

Materials useful in forming an erodible matrix include, but are not limited to, chitin, chitosan, dextran, and pullulan; gum agar, gum arabic, gum karaya, locust bean gum, gum tragacanth, carrageenans, gum ghatti, guar gum, xanthan gum, and scleroglucan; starches, such as dextrin and maltodextrin; hydrophilic colloids, such as pectin; phosphatides, such as lecithin; alginates; propylene glycol alginate; gelatin; collagen; and cellulosics, such as ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethylhydroxy ethylcellulose (EHEC); polyvinyl pyrrolidone; polyvinyl alcohol; polyvinyl acetate; glycerol fatty acid esters; polyacrylamide; polyacrylic acid; copolymers of ethacrylic acid or methacrylic acid (EUDRAGIT^{®}, Rohm America, Inc., Piscataway, NJ); poly(2-hydroxyethyl-methacrylate); polylactides; copolymers of L-glutamic acid and ethyl-L-glutamate; degradable lactic acidglycolic acid copolymers; poly-D-(-)-3-hydroxybutyric acid; and other acrylic acid derivatives, such as homopolymers and copolymers of butylmethacrylate, methylmethacrylate, ethylmethacrylate, ethyl acrylate, (2-dimethylaminoethyl)methacrylate, and (trimethylaminoethyl)methacrylate chloride.

In another embodiment, the pharmaceutical compositions are formulated with a non-erodible matrix device. The active ingredient(s) is dissolved or dispersed in an inert matrix and is released primarily by diffusion through the inert matrix once administered. Materials suitable for use as a non-erodible matrix device included, but are not limited to, insoluble plastics, such as polyethylene, polypropylene, polyisoprene, polyisobutylene, polybutadiene, polymethylmethacrylate, polybutylmethacrylate, chlorinated polyethylene, polyvinylchloride, methyl acrylate-methyl methacrylate copolymers, ethylene-vinylacetate copolymers, ethylene/propylene copolymers, ethylene/ethyl acrylate copolymers, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, polyvinyl chloride, plasticized nylon, plasticized polyethyleneterephthalate, natural rubber, silicone rubbers, polydimethylsiloxanes, silicone carbonate copolymers, and; hydrophilic polymers, such as ethyl cellulose, cellulose acetate, crospovidone, and cross-linked partially hydrolyzed polyvinyl acetate,; and fatty compounds, such as carnauba wax, microcrystalline wax, and triglycerides.

In a matrix controlled release system, the desired release kinetics can be controlled, for example, via the polymer type employed, the polymer viscosity, the particle sizes of the polymer and/or the active ingredient(s), the ratio of the active ingredient(s) versus the polymer, and other excipients in the compositions.

The pharmaceutical compositions provided herein in a modified release dosage form may be prepared by methods known to those skilled in the art, including direct compression, dry or wet granulation followed by compression, melt-granulation followed by compression.

### Osmotic Controlled Release Devices

The pharmaceutical compositions provided herein in a modified release dosage form may be fabricated using an osmotic controlled release device, including one-chamber system, two-chamber system, asymmetric membrane technology (AMT), and extruding core system (ECS). In general, such devices have at least two components: (a) the core which contains the active ingredient(s); and (b) a semipermeable membrane with at least one delivery port, which encapsulates the core. The semipermeable membrane controls the influx of water to the core from an aqueous environment of use so as to cause drug release by extrusion through the delivery port(s).

In addition to the active ingredient(s), the core of the osmotic device optionally includes an osmotic agent, which creates a driving force for transport of water from the environment of use into the core of the device. One class of osmotic agents waterswellable hydrophilic polymers, which are also referred to as "osmopolymers" and "hydrogels," including, but not limited to, hydrophilic vinyl and acrylic polymers, polysaccharides such as calcium alginate, polyethylene oxide (PEO), polyethylene glycol (PEG), polypropylene glycol (PPG), poly(2-hydroxyethyl methacrylate), poly(acrylic) acid, poly(methacrylic) acid, polyvinylpyrrolidone (PVP), crosslinked PVP, polyvinyl alcohol (PVA), PVA/PVP copolymers, PVA/PVP copolymers with hydrophobic monomers such as methyl methacrylate and vinyl acetate, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl, cellulose (CEC), sodium alginate, polycarbophil, gelatin, xanthan gum, and sodium starch glycolate.

The other class of osmotic agents is osmogens, which are capable of imbibing water to affect an osmotic pressure gradient across the barrier of the surrounding coating. Suitable osmogens include, but are not limited to, inorganic salts, such as magnesium sulfate, magnesium chloride, calcium chloride, sodium chloride, lithium chloride, potassium sulfate, potassium phosphates, sodium carbonate, sodium sulfite, lithium sulfate, potassium chloride, and sodium sulfate; sugars, such as dextrose, fructose, glucose, inositol, lactose, maltose, mannitol, raffinose, sorbitol, sucrose, trehalose, and xylitol,; organic acids, such as ascorbic acid, benzoic acid, fumaric acid, citric acid, maleic acid, sebacic acid, sorbic acid, adipic acid, edetic acid, glutamic acid, p-tolunesulfonic acid, succinic acid, and tartaric acid; urea; and mixtures thereof.

Osmotic agents of different dissolution rates may be employed to influence how rapidly the active ingredient(s) is initially delivered from the dosage form. For example, amorphous sugars, such as Mannogeme EZ (SPI Pharma, Lewes, DE) can be used to provide faster delivery during the first couple of hours to promptly produce the desired therapeutic effect, and gradually and continually release of the remaining amount to maintain the desired level of therapeutic or prophylactic effect over an extended period of time. In this case, the active ingredient(s) is released at such a rate to replace the amount of the active ingredient metabolized and excreted.

The core may also include a wide variety of other excipients and carriers as described herein to enhance the performance of the dosage form or to promote stability or processing.

Materials useful in forming the semipermeable membrane include various grades of acrylics, vinyls, ethers, polyamides, polyesters, and cellulosic derivatives that are water-permeable and water-insoluble at physiologically relevant pHs, or are susceptible to being rendered water-insoluble by chemical alteration, such as crosslinking. Examples of suitable polymers useful in forming the coating, include plasticized, unplasticized, and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate trimellitate (CAT), CA dimethylaminoacetate, CAethyl carbonate, CA chloroacetate, CA ethyl oxalate, CA methyl sulfonate, CA butyl sulfonate, CA p-toluene sulfonate, agar acetate, amylose triacetate, beta glucan acetate, beta glucan triacetate, acetaldehyde dimethyl acetate, triacetate of locust bean gum, hydroxlated ethylene-vinylacetate, EC, PEG, PPG, PEG/PPG copolymers, PVP, HEC, HPC, CMC, CMEC, HPMC, HPMCP, HPMCAS, HPMCAT, poly(acrylic) acids and esters and poly(methacrylic) acids and esters and copolymers thereof, starch, dextran, dextrin, chitosan, collagen, gelatin, polyalkenes, polyethers, polysulfones, polyethersulfones, polystyrenes, polyvinyl halides, polyvinyl esters and ethers, natural waxes, and synthetic waxes.

Semipermeable membrane may also be a hydrophobic microporous membrane, wherein the pores are substantially filled with a gas and are not wetted by the aqueous medium but are permeable to water, as disclosed in U.S. Pat. No. 5,798,119. Such hydrophobic but water- permeable membrane are typically composed of hydrophobic polymers such as polyalkenes, polyethylene, polypropylene, polytetrafluoroethylene, polyacrylic acid derivatives, polyethers, polysulfones, polyethersulfones, polystyrenes, polyvinyl halides, polyvinylidene fluoride, polyvinyl esters and ethers, natural waxes, and synthetic waxes.

The delivery port(s) on the semipermeable membrane may be formed postcoating by mechanical or laser drilling. Delivery port(s) may also be formed in situ by erosion of a plug of water-soluble material or by rupture of a thinner portion of the membrane over an indentation in the core. In addition, delivery ports may be formed during coating process, as in the case of asymmetric membrane coatings of the type disclosed in U.S. Pat. Nos. 5,612,059 and 5,698,220.

The total amount of the active ingredient(s) released and the release rate can substantially by modulated via the thickness and porosity of the semipermeable membrane, the composition of the core, and the number, size, and position of the delivery ports.

The pharmaceutical compositions in an osmotic controlled-release dosage form may further comprise additional conventional excipients as described herein to promote performance or processing of the formulation.

The osmotic controlled-release dosage forms can be prepared according to conventional methods and techniques known to those skilled in the art *(see,* Remington: The Science and Practice of Pharmacy*,* supra; Santus and Baker, J. Controlled Release 1995, 35, 1-21; Verma et al., Drug Development and Industrial Pharmacy 2000,26, 695-708; Verma et al., J. Controlled Release 2002, 79, 7-27).

In certain embodiments, the pharmaceutical compositions provided herein are formulated as AMT controlled-release dosage form, which comprises an asymmetric osmotic membrane that coats a core comprising the active ingredient(s) and other pharmaceutically acceptable excipients. *See,* U.S. Pat. No. 5,612,059 and WO 2002/17918. The AMT controlled-release dosage forms can be prepared according to conventional methods and techniques known to those skilled in the art, including direct compression, dry granulation, wet granulation, and a dip-coating method.

In certain embodiment, the pharmaceutical compositions provided herein are formulated as ESC controlled-release dosage form, which comprises an osmotic membrane that coats a core comprising the active ingredient(s), hydroxylethyl cellulose, and other pharmaceutically acceptable excipients.

### Multiparticulate Controlled Release Devices

The pharmaceutical compositions provided herein in a modified release dosage form may be fabricated a multiparticulate controlled release device, which comprises a multiplicity of particles, granules, or pellets, ranging from about 10 µm to about 3 mm, about 50 µm to about 2.5 mm, or from about 100 µm to 1 mm in diameter. Such multiparticulates may be made by the processes know to those skilled in the art, including wet-and dry-granulation, extrusion/spheronization, roller-compaction, melt-congealing, and by spray-coating seed cores. *See,* for example, Multiparticulate Oral Drug Delivery; Marcel Dekker: 1994; and Pharmaceutical Pelletization Technology; Marcel Dekker: 1989.

Other excipients as described herein may be blended with the pharmaceutical compositions to aid in processing and forming the multiparticulates. The resulting particles may themselves constitute the multiparticulate device or may be coated by various filmforming materials, such as enteric polymers, water-swellable, and water-soluble polymers. The multiparticulates can be further processed as a capsule or a tablet.

### Targeted Delivery

The pharmaceutical compositions provided herein may also be formulated to be targeted to a particular tissue, receptor, or other area of the body of the subject to be treated, including liposome-, resealed erythrocyte-, and antibody-based delivery systems. Examples include, but are not limited to, U.S. Pat. Nos. 6,316,652; 6,274,552; 6,271,359; 6,253,872; 6,139,865; 6,131,570; 6,120,751; 6,071,495; 6,060,082; 6,048,736; 6,039,975; 6,004,534; 5,985,307; 5,972,366; 5,900,252; 5,840,674; 5,759,542; and 5,709,874.

### Dosages

In the treatment, prevention, or amelioration of one or more symptoms of schizophrenia or schizoaffective disorder or other conditions, disorders or diseases associated with VMAT2 inhibition, an appropriate dosage level generally is about 0.001 to 100 mg per kg patient body weight per day (mg/kg per day), about 0.01 to about 80 mg/kg per day, about 0.1 to about 50 mg/kg per day, about 0.5 to about 25 mg/kg per day, or about 1 to about 20 mg/kg per day, which may be administered in single or multiple doses. Within this range the dosage may be 0.005 to 0.05, 0.05 to 0.5, or 0.5 to 5.0, 1 to 15, 1 to 20, or 1 to 50 mg/kg per day. In certain embodiments, the dosage level is about 0.001 to 100 mg/kg per day. In certain embodiments, the dosage level is about from 5.0 to 150 mg per day, and in certain embodiments from 10 to 100 mg per day. In other embodiments, the dosage level is about from 25 to 100 mg/kg per day. In certain embodiments, the dosage level is about 0.01 to about 40 mg/kg per day. In certain embodiments, the dosage level is about 0.1 to about 80 mg/kg per day. In certain embodiments, the dosage level is about 0.1 to about 50 mg/kg per day. In certain embodiments, the dosage level is about 0.1 to about 40 mg/kg per day. In certain embodiments, the dosage level is about 0.5 to about 80 mg/kg per day. In certain embodiments, the dosage level is about 0.5 to about 40 mg/kg per day. In certain embodiments, the dosage level is about 0.5 to about 25 mg/kg per day. In certain embodiments, the dosage level is about 1 to about 80 mg/kg per day. In certain embodiments, the dosage level is about 1 to about 75 mg/kg per day. In certain embodiments, the dosage level is about 1 to about 50 mg/kg per day. In certain embodiments, the dosage level is about 1 to about 40 mg/kg per day. In certain embodiments, the dosage level is about 1 to about 25 mg/kg per day. In certain embodiments, the dosage level is about 80 mg per day. In certain embodiments, the dosage level is about 40 mg per day.

For oral administration, the pharmaceutical compositions can be provided in the form of tablets containing 1.0 to 1,000 mg of the active ingredient, particularly about 1, about 5, about 10, about 15, about 20, about 25, about 30, about 40, about 45, about 50, about 75, about 80, about 100, about 150, about 200, about 250, about 300, about 400, about 500, about 600, about 750, about 800, about 900, and about 1,000 mg of the active ingredient for the symptomatic adjustment of the dosage to the patient to be treated. In certain embodiments, the pharmaceutical compositions can be provided in the form of tablets containing about 100 mg of the active ingredient. In certain embodiments, the pharmaceutical compositions can be provided in the form of tablets containing about 80 mg of the active ingredient. In certain embodiments, the pharmaceutical compositions can be provided in the form of tablets containing about 75 mg of the active ingredient. In certain embodiments, the pharmaceutical compositions can be provided in the form of tablets containing about 50 mg of the active ingredient. In certain embodiments, the pharmaceutical compositions can be provided in the form of tablets containing about 40 mg of the active ingredient. In certain embodiments, the pharmaceutical compositions can be provided in the form of tablets containing about 25 mg of the active ingredient. The compositions may be administered on a regimen of 1 to 4 times per day, including once, twice, three times, and four times per day.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

Also provided herein are methods of modulating VMAT2 activity, comprising contacting the transporter with the compounds in one or more solid forms as provided herein. In one embodiment, the transporter is expressed by a cell.

The compounds provided herein may also be combined or used in combination with other agents useful in the treatment, prevention, or amelioration of one or more symptoms of the diseases or conditions for which the compounds provided herein are useful, including schizophrenia or schizoaffective disorder and other conditions commonly treated with antipsychotic medication.

In one embodiment, the compounds provided herein may also be combined or used in combination with a typical antipsychotic drug. In specific embodiments, the typical antipsychotic drug is fluphenazine, haloperidol, loxapine, molindone, perphenazine, pimozide, sulpiride, thioridazine, or trifluoperazine. In other particular embodiments, the antipsychotic drug is an atypical antipsychotic drug. In more specific embodiments, the atypical antipsychotic drug is aripiprazole, asenapine, clozapine, iloperidone, olanzapine, paliperidone, quetiapine, risperidone, or ziprasidone. In one particular embodiment, the atypical antipsychotic drug is clozapine.

Such other agents, or drugs, may be administered, by a route and in an amount commonly used thereof, simultaneously or sequentially with the compounds provided herein. When an the particulates provided herein are used contemporaneously with one or more other drugs, a pharmaceutical composition containing such other drugs in addition to the compounds provided herein may be utilized, but is not required.· Accordingly, the pharmaceutical compositions provided herein include those that also contain one or more other active ingredients or therapeutic agents, in addition to the compounds provided herein.

The weight ratio of the compounds provided herein to the second active ingredient may be varied, and will depend upon the effective dose of each ingredient. Generally, an effective dose of each will be used. Thus, for example, when the compounds provided herein are used in combination with the second drug, or a pharmaceutical composition containing such other drug, the weight ratio of the particulates to the second drug may range from about 1,000:1 to about 1: 1,000, or about 200:1 to about 1:200. Combinations of the particulates provided herein and other active ingredients will generally also be within the aforementioned range, but in each case, an effective dose of each active ingredient should be used.

### Pharmakokinetic Properties

In certain embodiments, (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof, is metabolized *in vivo* to its active form, (2R, 3R, 11b*R*)-3-isobutyl-9,10-dimethoxy-1,3,4,6,7,11b-hexahydro-2H-pyrido[2,1-a]isoquinolin-2-ol, also known as dihydrotetrabenazine: (+)α-DHTBZ, which is believed to be the most active metabolite *(see, e.g.,* Kilbourn *et al. Chirality,* 1997, 9, 59-62).

In one embodiment, a method for treating schizophrenia or schizoaffective disorder is provided herein that comprises administering to a subject in need thereof a pharmaceutical composition comprising (*S*)-2-amino-3-methyl-butyric acid (2*R*,3*R*,11b*R)*-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof, in an amount sufficient to achieve a maximal blood plasma concentration (Cₘₐₓ) of (2*R*,3*R*,11b*R*)-3-isobutyl-9,10-dimethoxy-1,3,4,6,7,11b-hexahydro-2H-pyrido[2,1-a]isoquinolin-2-ol *(R, R, R* DHTBZ) of between about 15 ng to about 60 ng per mL plasma and a minimal blood plasma concentration (Cₘᵢₙ) of (2*R*,*3*R,11b*R*)-3-isobutyl-9,10-dimethoxy-1,3,4,6,7,11b-hexahydro-2H-pyrido[2,1-a]isoquinolin-2-ol *(R, R, R* DHTBZ) of at least 15 ng per mL plasma over an 8 hour period.

In other embodiments, reference to plasma concentration of (2*R*,3*R*,11b*R*)-3-isobutyl-9,10-dimethoxy-1,3,4,6,7,1 1b-hexahydro-2H-pyrido[2,1-a]isoquinolin-2-ol *(R, R, R* DHTBZ) in the methods described herein includes both deuterated (2*R*,3*R*,1b*R*)-3-isobutyl-9,10-dimethoxy-1,3,4,6,7,11b-hexahydro-2H-pyrido[2,1-a]isoquinolin-2-ol *(R, R, R* DHTBZ) and non-deuterated (2*R*,3*R*,11b*R*)-3-isobutyl-9,10-dimethoxy-1,3,4,6,7,11b-hexahydro-2H-pyrido[2,1-a]isoquinolin-2-ol *(R, R, R* DHTBZ). It is apparent to a person of skill in the art that if a deuterated VMAT2 inhibitor as described herein is administered to a subject (*e.g.*, deuterated (*S*)-2-amino-3-methyl-butyric acid (2*R*,3*R*,11b*R*)-3-isobutyl-9,10-dimethoxy-1,3,4,6,7,11b-hexahydro-2H-pyrido[2,l-a]isoquinolin-2-yl ester, or deutereted (2*R*,3*R*,11b*R*)-3-isobutyl-9,10-dimethoxy-1,3,4,6,7,11b-hexahydro-2H-pyrido[2,1-a]isoquinolin-2-ol), then deuterated (2*R*,3*R*,11b*R*)-3-isobutyl-9, 10-dimethoxy-1,3,4,6,7,11b-hexahydro-2H-pyrido[2,1-a]isoquinolin-2-ol will appear in the subject's blood plasma and is to be measured. If a non-deuterated VMAT2 inhibitor as described herein is administered to a subject (e.g., non-deuterated (*S*)-2-amino-3-methyl-butyric acid (2*R*,3*R*,11b*R*)-3-isobutyl-9,10-dimethoxy-1,3,4,6,7,1 1b-hexahydro-2H-pyrido[2,l-a]isoquinolin-2-yl ester, or non-deutereted (2*R*,3*R*,11b*R*)-3-isobutyl-9,10-dimethoxy-1,3,4,6,7,11b-hexahydro-2H-pyrido[2,1-a]isoquinolin-2-ol), then non-deuterated (2*R*,3*R*,11b*R*)-3-isobuty1-9,10-dimethoxy-1,3,4,6,7,1 1b-hexahydro-2H-pyrido[2,1-a]isoquinolin-2-ol will appear in the subject's blood plasma and is to be measured. If a combination of deuterated and non-deuterated VMAT2 inhibitors as described herein is administered to a subject, then both deuterated and non-deuterated (2*R*,3*R*,11b*R*)-3-isobutyl-9, 10-dimethoxy-1,3,4,6,7,11b-hexahydro-2H-pyrido[2,1-a]isoquinolin-2-ol will appear in the subject's blood plasma and both are to be measured.

In certain embodiments, the Cₘₐₓ of (2*R*,3*R*,11b*R*)-3-isobutyl-9,10-dimethoxy-1,3,4,6,7,1 1b-hexahydro-2H-pyrido[2,1-a]isoquinolin-2-ol *(R, R, R* DHTBZ) is about 15 ng/mL, about 20 ng/mL, about 25 ng/mL, about 30 ng/mL, about 35 ng/mL, about 40 ng/mL, about 45 ng/mL, about 50 ng/mL, about 55 ng/mL or about 60 ng/mL plasma. In certain embodiments, the Cₘᵢₙ (2*R*,3*R*,11b*R*)-3-isobutyl-9,10-dimethoxy-1,3,4,6,7,11b-hexahydro-2H-pyrido[2,1-a]isoquinolin-2-ol *(R, R, R* DHTBZ) is at least 15 ng/mL, at least 20 ng/mL, at least 25 ng/mL, at least 30 ng/mL, or at least 35 ng/mL plasma, over a period of 8 hrs, 12 hrs, 16 hrs, 20 hrs, 24 hrs, 28 hrs, or 32 hrs. In certain embodiments, the Cₘᵢₙ of (2*R*,3*R*,11b*R*)-3-isobutyl-9,10-dimethoxy-1,3,4,6,7,1 1b-hexahydro-2H-pyrido[2,1-a]isoquinolin-2-ol (*R, R, R* DHTBZ)is between about 15 ng/mL to about 35 ng/mL.

In one embodiment, the pharmaceutical composition is administered in an amount sufficient to provide a Cₘₐₓ of (2*R*,3*R*,11b*R*)-3-isobutyl-9,10-dimethoxy-1,3,4,6,7,11b-hexahydro-2H-pyrido[2,l-a]isoquinolin-2-ol (*R, R, R* DHTBZ) of about 15 ng/mL to about 60 ng/mL plasma and a Cₘᵢₙ of approximately at least 33% of the Cₘₐₓ over a 24 hour period. In another embodiment, the pharmaceutical composition is administered in an amount sufficient to provide a Cₘₐₓ of (2*R*,3*R*,11b*R*)-3-isobutyl-9,10-dimethoxy-1,3,4,6,7,11b-hexahydro-2H-pyrido[2,1-a]isoquinolin-2-ol (*R, R, R* DHTBZ) of about 15 ng/mL to about 60 ng/mL plasma and a Cₘᵢₙ of approximately at least 50% of the Cₘₐₓ over a 24 hour period. In certain particular embodiments, the pharmaceutical composition is administered in an amount sufficient to provide a Cₘₐₓ of (2*R*,3*R*,11b*R*)-3-isobutyl-9,10-dimethoxy-1,3,4,6,7,11b-hexahydro-2H-pyrido[2,l-a]isoquinolin-2-ol (*R, R, R* DHTBZ) of about 15 ng/mL to about 60 ng/mL plasma and a Cₘᵢₙ of approximately between about at least 33% -50% of the Cₘₐₓ over a 24 hour period.

In certain embodiments, the pharmaceutical composition is administered in an amount sufficient to provide a Cₘₐₓ of (2*R*,3*R*,11*b*R)-3-isobutyl-9,10-dimethoxy-1,3,4,6,7, 1 1b-hexahydro-2H-pyrido[2,1-a]isoquinolin-2-ol *(R, R, R* DHTBZ) of about 15 ng/mL to about 60 ng/mL plasma and a Cₘᵢₙ of approximately at least 33% of the Cₘₐₓ over a 12 hour period. In yet another certain embodiment, the pharmaceutical composition is administered in an amount sufficient to provide a Cₘₐₓ of (2*R*,3*R*,11bR)-3-isobutyl-9, 10-dimethoxy-1,3,4,6,7, 1 1b-hexahydro-2H-pyrido[2,1-a]isoquinolin-2-ol *(R, R, R* DHTBZ) of about 15 ng/mL to about 60 ng/mL plasma and a Cₘᵢₙ of approximately at least 50% of the Cₘₐₓ over a 12 hour period. In certain particular embodiments, the pharmaceutical composition is administered in an amount sufficient to provide a Cₘₐₓ of (2*R*,3*R*,11b*R*)-3-isobutyl-9,10-dimethoxy-1,3,4,6,7,11b-hexahydro-2H-pyrido[2,1-a]isoquinolin-2-ol *(R, R, R* DHTBZ) of about 15 ng/mL to about 60 ng/mL plasma and a Cₘᵢₙ of approximately between about at least 33% -50% of the Cₘₐₓ over a 12 hour period.

In another embodiment, the pharmaceutical composition is administered to a subject in need thereof in an amount that provides a Cₘₐₓ of (2*R*,3*R*,11b*R*)-3-isobutyl-9,10-dimethoxy-1,3,4,6,7, 1 1b-hexahydro-2H-pyrido[2,1-a]isoquinolin-2-ol *(R, R, R* DHTBZ) of about 15 ng/mL to about 60 ng/mL plasma and a Cₘᵢₙ of between about 5 ng/mL to about 30 ng/mL plasma over a 24 hour period. In yet another embodiment, the pharmaceutical composition is administered to a subject in need thereof in an amount that provides a Cₘₐₓ of (2*R*,3*R*,11b*R*)-3-isobutyl-9,10-dimethoxy-1,3,4,6,7,11b-hexahydro-2H-pyrido[2,1-a]isoquinolin-2-ol *(R, R, R* DHTBZ) of about 15 ng/mL to about 60 ng/mL plasma and a Cₘᵢₙ of between about 7.5 ng/mL to about 30 ng/mL plasma over a 24 hour period.

In another embodiment, a method for treating schizophrenia or schizoaffective disorder is provided herein that comprises administering to a subject in need thereof a pharmaceutical composition comprising (*S*)-2-amino-3-methyl-butyric acid (2*R*,3*R*,11b*R*)-3-isobutyl-9,10-dimethoxy-1,3,4,6,7, 1 1b-hexahydro-2H-pyrido[2,l-a]isoquinolin-2-yl ester, or an isotopic variant thereof; or a pharmaceutically acceptable salt, or polymorph thereof, as an active pharmaceutical ingredient, in an amount sufficient to provide: (i) a therapeutic concentration range of about 15 ng to about 60 ng of (2*R*,3*R*,11bR)-3-isobutyl-9, 10-dimethoxy-1,3,4,6,7,11b-hexahydro-2H-pyrido[2,1-a]isoquinolin-2-ol *(R, R, R* DHTBZ) per mL plasma; and (ii) a threshold concentration of at least 15 ng (2*R*,3*R*,11b*R*)-3-isobutyl-9,10-dimethoxy-1,3,4,6,7,11b-hexahydro-2H-pyrido[2,1-a]isoquinolin-2-ol *(R, R, R* DHTBZ) per mL plasma over a period of about 8 hours to about 24 hours.

In certain embodiments, the therapeutic concentration range is about 15 ng to about 35 ng, to about 40 ng, to about 45 ng, to about 50 ng, or to about 55 ng (2*R*,3*R*,11b*R*)-3-isobutyl-9,10-dimethoxy-1,3,4,6,7,1 1b-hexahydro-2H-pyrido[2,1-a]isoquinolin-2-ol *(R, R, R* DHTBZ) per mL plasma.

In certain embodiments, the threshold concentration of (2*R*,3*R*,11b*R*)-3-isobutyl-9,10-dimethoxy-1,3,4,6,7,11b-hexahydro-2H-pyrido[2,1-a]isoquinolin-2-ol (*R, R, R* DHTBZ) is about 15 ng/mL, about 20 ng/mL, about 25 ng/mL, about 30 ng/mL, about 35 ng/mL, about 40 ng/mL, about 45 ng/mL, about 50 ng/mL, about 55 ng/mL or about 60 ng/mL plasma, over a period of about 8 hrs, about 12 hrs, about 16 hrs, about 20 hrs, about 24 hrs, about 28 hrs, or about 32 hrs. In a particular embodiment, the threshold concentration of (2*R*,3*R*,11b*R*)-3-isobutyl-9,10-dimethoxy-1,3,4,6,7,11b-hexahydro-2H-pyrido[2,1-a]isoquinolin-2-ol (R, R, R DHTBZ) is between about 15 ng/mL to about 35 ng/mL over a period of about 8 hours to about 24 hours.

Plasma concentrations of (2*R*,3*R*,11bR)-3-isobutyl-9,10-dimethoxy-1,3,4,6,7,11b-hexahydro-2H-pyrido[2,1-a]isoquinolin-2-ol, and compounds as disclosed herein may be measured by methods as described in Derangula et al., Biomedical Chromatography 2013 27(6): 792-801, Mehvar et al., Drug Metabolism and Distribution 1987 15(2): 250-55 and generally by tandem mass spectroscopy.

These and other changes can be made to the embodiments in light of the above-detailed description. Although specific embodiments have been described herein for purposes of illustration, various modifications of the above-described modes for carrying out the disclosure that are obvious to persons of skill in the art are intended to be within the scope of the following claims. All publications, patents, and patent applications cited in this specification are incorporated herein by reference as if each such publication, patent, or patent application were specifically and individually indicated to be incorporated herein by reference.

The following numbered paragraphs set out preferred features and preferred combinations of features of the present invention:
1. A method of treating schizophrenia or schizoaffective disorder, comprising administering (S)-2-amino-3-methyl-butyric acid (2R,3R,11bR)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof.
2. A method of treating behavioral problems associated with schizophrenia or schizoaffective disorder, comprising administering (S)-2-amino-3-methyl-butyric acid (2R,3R,11bR)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof.
3. The method of paragraphs 1 or 2, wherein the (S)-2-amino-3-methyl-butyric acid (2R,3R,11bR)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof, is (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.
4. The method of any of paragraphs 1 to 3, wherein the method treats positive symptoms.
5. The method of any of paragraphs 1 to 3, wherein the method treats negative symptoms.
6. The method of any of paragraphs 1 to 5, wherein (S)-2-amino-3-methyl-butyric acid (2R,3R,11bR)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof, is in the form of a dosage unit.
7. The method of paragraph 6, wherein the dosage unit contains 25 to 100 mg of (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.
8. The method of paragraph 7, wherein the dosage unit contains 40 mg of (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.
9. The method of paragraph 8, wherein the dosage unit contains 80 mg of (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.
10. The method of any of paragraphs 6 to 8, wherein the dosage unit is a tablet or capsule.
11. The compound (S)-2-amino-3-methyl-butyric acid (2R,3R,11bR)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof, for use in treating schizophrenia or schizoaffective disorder.
12. The compound for use of paragraph 11, wherein the compound is formulated for oral administration.
13. The compound for use of paragraphs 11 or 12, wherein the compound is formulated as a single dosage form.
14. The compound for use of paragraph 13, wherein the dosage form is a tablet or capsule.
15. The compound for use of paragraph 13 or 14, wherein the dosage form contains 40 mg of (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.
16. The compound for use of paragraph 13 or 14, wherein the dosage form contains 80 mg of (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.

## Claims

1. A method of treating schizophrenia or schizoaffective disorder, comprising administering (S)-2-amino-3-methyl-butyric acid (2R,3R,11bR)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof.

2. A method of treating behavioral problems associated with schizophrenia or schizoaffective disorder, comprising administering (S)-2-amino-3-methyl-butyric acid (2R,3R,11bR)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof.

3. The method of claims 1 or 2, wherein the (S)-2-amino-3-methyl-butyric acid (2R,3R,11bR)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof, is (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.

4. The method of any of claims 1 to 3, wherein the method treats positive symptoms.

5. The method of any of claims 1 to 3, wherein the method treats negative symptoms.

6. The method of any of claims 1 to 5, wherein (S)-2-amino-3-methyl-butyric acid (2R,3R,11bR)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof, is in the form of a dosage unit.

7. The method of claim 6, wherein the dosage unit contains 25 to 100 mg of (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.

8. The method of claim 7, wherein the dosage unit contains 40 mg of (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.

9. The method of claim 8, wherein the dosage unit contains 80 mg of (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.

10. The method of any of claims 6 to 8, wherein the dosage unit is a tablet or capsule.

11. The compound (S)-2-amino-3-methyl-butyric acid (2R,3R,11bR)-3-isobutyl-9,10-dimethoxy-1,3,4,6,7,11b-hexahydro-2H-pyrido[2,l-a]isoquinolin-2-yl ester, or an isotopic variant thereof, or a pharmaceutically acceptable salt, or polymorph thereof, for use in treating schizophrenia or schizoaffective disorder.

12. The compound for use of claim 11, wherein the compound is formulated for oral administration.

13. The compound for use of claims 11 or 12, wherein the compound is formulated as a single dosage form.

14. The compound for use of claim 13, wherein the dosage form contains 40 mg of (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.

15. The compound for use of claim 13 or 14, wherein the dosage form contains 80 mg of (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate di(4-methylbenzenesulfonate), or an isotopic variant thereof, or polymorph thereof.
